# EUROPEAN PATENT SPECIFICATION

(11) **EP 3 003 366 B1**
(45) Date of publication and mention of the grant of the patent: **05.08.2020**
(21) Application number: 14792563.0
(22) Date of filing: 06.06.2014
(51) Int. Cl.: A61K 39/36, C07K 14/415, A61K 39/35, C07K 14/435, A61K 39/02, A61K 39/00

(54) **CONTIGUOUS OVERLAPPING PEPTIDES FOR TREATMENT OF HOUSE DUST MITES ALLERGY**
BENACHBARTE ÜBERLAPPENDE PEPTIDE ZUR BEHANDLUNG VON HAUSSTAUBMILBENALLERGIE
PEPTIDES CONTIGUS SE CHEVAUCHANT POUR LE TRAITEMENT D'UNE ALLERGIE AUX ACARIENS DE LA POUSSIÈRE

(30) Priority: 06.06.2013 US 201361831961 P
(43) Date of publication of application: 13.04.2016
(73) Proprietor: Anergis S.A., 1066 Epalinges (CH)
(72) Inventor: KETTNER, Alexander, CH-1012 Lausanne (CH); REYMOND, Christophe, CH-1008 Prilly (CH)
(74) Representative: Zwicker, Jörk
(86) International application number: PCT/IB2014/001888
(87) International publication number: WO 2014/195803

(56) References cited:
- WO-A2-2004/081028
- WO-A2-2010/089671
- WO-A2-2013/001362
- US-B1- 6 268 491
- J. A. ASTURIAS ET AL: "Engineering of major house dust mite allergens Der p 1 and Der p 2 for allergen-specific immunotherapy", CLINICAL & EXPERIMENTAL ALLERGY, vol. 39, no. 7, 1 July 2009 (2009-07-01), pages 1088-1098, XP055169215, ISSN: 0954-7894, DOI: 10.1111/j.1365-2222.2009.03264.x
- DEREWENDA U ET AL: "The crystal structure of a major dust mite allergen Der p 2, and its biological implications", JOURNAL OF MOLECULAR BIOLOGY, ACADEMIC PRESS, UNITED KINGDOM, vol. 318, no. 1, 19 April 2002 (2002-04-19), pages 189-197, XP002326694, ISSN: 0022-2836, DOI: 10.1016/S0022-2836(02)00027-X
- KUAN-WEI CHEN ET AL: "Hypoallergenic Der p 1/Der p 2 combination vaccines for immunotherapy of house dust mite allergy", JOURNAL OF ALLERGY AND CLINICAL IMMUNOLOGY, ELSEVIER, AMSTERDAM, NL, vol. 130, no. 2, 23 May 2012 (2012-05-23), pages 435-443.e4, XP028431297, ISSN: 0091-6749, DOI: 10.1016/J.JACI.2012.05.035 [retrieved on 2012-06-02]
- VRTALA S ET AL: "Conversion of the major birch pollen allergen, bet v 1, into two nonanaphylactic T cell epitope-containing fragments: Candidates for a novel form of specific immunotherapy", JOURNAL OF CLINICAL INVESTIGATION, AMERICAN SOCIETY FOR CLINICAL INVESTIGATION, US, vol. 99, no. 7, 1 January 1997 (1997-01-01), pages 1673-1681, XP002221492, ISSN: 0021-9738, DOI: 10.1172/JCI119330
- GARNIER VON C ET AL: "Allergen-derived long peptide immunotherapy down-regulates specific IgE response and protects from anaphylaxis", EUROPEAN JOURNAL OF IMMUNOLOGY, WILEY - V C H VERLAG GMBH & CO. KGAA, DE, vol. 30, no. 6, 1 June 2000 (2000-06-01), pages 1638-1645, XP002297947, ISSN: 0014-2980, DOI: 10.1002/1521-4141(200006)30:6<1638::AID-IM MU1638>3.0.CO;2-R

### SEQUENCE LISTING

<110> Anergis SA
   Kettner, et al.
<120> CONTIGUOUS OVERLAPPING PEPTIDES FOR TREATMENT OF HOUSE DUST MITES
<130> 30985/47712A
<150> 61/831,961
   <151> 2013-06-06
<160> 29
<170> PatentIn version 3.5
<210> 1
   <211> 81
   <212> PRT
   <213> Dermatophagoides pteronyssinus
<400> 1
   Thr Asn Ala Cys Ser Ile Asn Gly Asn Ala Pro Ala Glu Ile Asp Leu
   1               5                   10                  15
   Arg Gln Met Arg Thr Val Thr Pro Ile Arg Met Gln Gly Gly Cys Gly
               20                  25                  30
   Ser Cys Trp Ala Phe Ser Gly Val Ala Ala Thr Glu Ser Ala Tyr Leu
           35                  40                  45
   Ala Tyr Arg Asn Gln Ser Leu Asp Leu Ala Glu Gln Glu Leu Val Asp
       50                  55                  60
   Cys Ala Ser Gln His Gly Cys His Gly Asp Thr Ile Pro Arg Gly Ile
   65                  70                  75                  80
   Glu
<210> 2
   <211> 86
   <212> PRT
   <213> Dermatophagoides pteronyssinus
<400> 2
   Ser Gln His Gly Cys His Gly Asp Thr Ile Pro Arg Gly Ile Glu Tyr
   1               5                   10                  15
   Ile Gln His Asn Gly Val Val Gln Glu Ser Tyr Tyr Arg Tyr Val Ala
               20                  25                  30
   Arg Glu Gln Ser Cys Arg Arg Pro Asn Ala Gln Arg Phe Gly Ile Ser
           35                  40                  45
   Asn Tyr Cys Gln Ile Tyr Pro Pro Asn Val Asn Lys Ile Arg Glu Ala
       50                  55                  60
   Leu Ala Gln Thr His Ser Ala Ile Ala Val Ile Ile Gly Ile Lys Asp
   65                  70                  75                  80
   Leu Asp Ala Phe Arg His
                   85
<210> 3
   <211> 86
   <212> PRT
   <213> Dermatophagoides pteronyssinus
<400> 3
   Ala Ile Ala Val Ile Ile Gly Ile Lys Asp Leu Asp Ala Phe Arg His
   1               5                   10                  15
   Tyr Asp Gly Arg Thr Ile Ile Gln Arg Asp Asn Gly Tyr Gln Pro Asn
               20                  25                  30
   Tyr His Ala Val Asn Ile Val Gly Tyr Ser Asn Ala Gln Gly Val Asp
           35                  40                  45
   Tyr Trp Ile Val Arg Asn Ser Trp Asp Thr Asn Trp Gly Asp Asn Gly
       50                  55                  60
   Tyr Gly Tyr Phe Ala Ala Asn Ile Asp Leu Met Met Ile Glu Glu Tyr
   65                  70                  75                  80
   Pro Tyr Val Val Ile Leu
                   85
<210> 4
   <211> 66
   <212> PRT
   <213> Dermatophagoides pteronyssinus
<400> 4
   Thr Asn Ala Cys Ser Ile Asn Gly Asn Ala Pro Ala Glu Ile Asp Leu
   1               5                   10                  15
   Arg Gln Met Arg Thr Val Thr Pro Ile Arg Met Gln Gly Gly Cys Gly
               20                  25                  30
   Ser Cys Trp Ala Phe Ser Gly Val Ala Ala Thr Glu Ser Ala Tyr Leu
           35                  40                  45
   Ala Tyr Arg Asn Gln Ser Leu Asp Leu Ala Glu Gln Glu Leu Val Asp
       50                  55                  60
   Cys Ala
   65
<210> 5
   <211> 73
   <212> PRT
   <213> Dermatophagoides pteronyssinus
<400> 5
   Leu Ala Glu Gln Glu Leu Val Asp Cys Ala Ser Gln His Gly Cys His
   1               5                   10                  15
   Gly Asp Thr Ile Pro Arg Gly Ile Glu Tyr Ile Gln His Asn Gly Val
               20                  25                  30
   Val Gln Glu Ser Tyr Tyr Arg Tyr Val Ala Arg Glu Gln Ser Cys Arg
           35                  40                  45
   Arg Pro Asn Ala Gln Arg Phe Gly Ile Ser Asn Tyr Cys Gln Ile Tyr
       50                  55                  60
   Pro Pro Asn Val Asn Lys Ile Arg Glu
   65                  70
<210> 6
   <211> 73
   <212> PRT
   <213> Dermatophagoides pteronyssinus
<400> 6
   Pro Asn Val Asn Lys Ile Arg Glu Ala Leu Ala Gln Thr His Ser Ala
   1               5                   10                  15
   Ile Ala Val Ile Ile Gly Ile Lys Asp Leu Asp Ala Phe Arg His Tyr
               20                  25                  30
   Asp Gly Arg Thr Ile Ile Gln Arg Asp Asn Gly Tyr Gln Pro Asn Tyr
           35                  40                  45
   His Ala Val Asn Ile Val Gly Tyr Ser Asn Ala Gln Gly Val Asp Tyr
       50                  55                  60
   Trp Ile Val Arg Asn Ser Trp Asp Thr
   65                  70
<210> 7
   <211> 40
   <212> PRT
   <213> Dermatophagoides pteronyssinus
<400> 7
   Val Asp Tyr Trp Ile Val Arg Asn Ser Trp Asp Thr Asn Trp Gly Asp
   1               5                   10                  15
   Asn Gly Tyr Gly Tyr Phe Ala Ala Asn Ile Asp Leu Met Met Ile Glu
               20                  25                  30
   Glu Tyr Pro Tyr Val Val Ile Leu
           35                  40
<210> 8
   <211> 70
   <212> PRT
   <213> Dermatophagoides pteronyssinus
<400> 8
   Thr Asn Ala Cys Ser Ile Asn Gly Asn Ala Pro Ala Glu Ile Asp Leu
   1               5                   10                  15
   Arg Gln Met Arg Thr Val Thr Pro Ile Arg Met Gln Gly Gly Cys Gly
               20                  25                  30
   Ser Cys Trp Ala Phe Ser Gly Val Ala Ala Thr Glu Ser Ala Tyr Leu
           35                  40                  45
   Ala Tyr Arg Asn Gln Ser Leu Asp Leu Ala Glu Gln Glu Leu Val Asp
       50                  55                  60
   Cys Ala Ser Gln His Gly
   65                  70
<210> 9
   <211> 64
   <212> PRT
   <213> Dermatophagoides pteronyssinus
<400> 9
   Ala Ser Gln His Gly Cys His Gly Asp Thr Ile Pro Arg Gly Ile Glu
   1               5                   10                  15
   Tyr Ile Gln His Asn Gly Val Val Gln Glu Ser Tyr Tyr Arg Tyr Val
               20                  25                  30
   Ala Arg Glu Gln Ser Cys Arg Arg Pro Asn Ala Gln Arg Phe Gly Ile
           35                  40                  45
   Ser Asn Tyr Cys Gln Ile Tyr Pro Pro Asn Val Asn Lys Ile Arg Glu
       50                  55                  60
<210> 10
   <211> 63
   <212> PRT
   <213> Dermatophagoides pteronyssinus
<400> 10
   Pro Asn Val Asn Lys Ile Arg Glu Ala Leu Ala Gln Thr His Ser Ala
   1               5                   10                  15
   Ile Ala Val Ile Ile Gly Ile Lys Asp Leu Asp Ala Phe Arg His Tyr
               20                  25                  30
   Asp Gly Arg Thr Ile Ile Gln Arg Asp Asn Gly Tyr Gln Pro Asn Tyr
           35                  40                  45
   His Ala Val Asn Ile Val Gly Tyr Ser Asn Ala Gln Gly Val Asp
       50                  55                  60
<210> 11
   <211> 43
   <212> PRT
   <213> Dermatophagoides pteronyssinus
<400> 11
   Ala Gln Gly Val Asp Tyr Trp Ile Val Arg Asn Ser Trp Asp Thr Asn
   1               5                   10                  15
   Trp Gly Asp Asn Gly Tyr Gly Tyr Phe Ala Ala Asn Ile Asp Leu Met
               20                  25                  30
   Met Ile Glu Glu Tyr Pro Tyr Val Val Ile Leu
           35                  40
<210> 12
   <211> 37
   <212> PRT
   <213> Dermatophagoides pteronyssinus
<400> 12
   Ala Ser Gln His Gly Cys His Gly Asp Thr Ile Pro Arg Gly Ile Glu
   1               5                   10                  15
   Tyr Ile Gln His Asn Gly Val Val Gln Glu Ser Tyr Tyr Arg Tyr Val
               20                  25                  30
   Ala Arg Glu Gln Ser
       35
<210> 13
   <211> 35
   <212> PRT
   <213> Dermatophagoides pteronyssinus
<400> 13
   Arg Tyr Val Ala Arg Glu Gln Ser Cys Arg Arg Pro Asn Ala Gln Arg
   1               5                   10                  15
   Phe Gly Ile Ser Asn Tyr Cys Gln Ile Tyr Pro Pro Asn Val Asn Lys
               20                  25                  30
   Ile Arg Glu
           35
<210> 14
   <211> 35
   <212> PRT
   <213> Dermatophagoides pteronyssinus
<400> 14
   Pro Asn Val Asn Lys Ile Arg Glu Ala Leu Ala Gln Thr His Ser Ala
   1               5                   10                  15
   Ile Ala Val Ile Ile Gly Ile Lys Asp Leu Asp Ala Phe Arg His Tyr
               20                  25                  30
   Asp Gly Arg
           35
<210> 15
   <211> 34
   <212> PRT
   <213> Dermatophagoides pteronyssinus
<400> 15
   Arg His Tyr Asp Gly Arg Thr Ile Ile Gln Arg Asp Asn Gly Tyr Gln
   1               5                   10                  15
   Pro Asn Tyr His Ala Val Asn Ile Val Gly Tyr Ser Asn Ala Gln Gly
               20                  25                  30
   Val Asp
<210> 16
   <211> 72
   <212> PRT
   <213> Dermatophagoides pteronyssinus
<400> 16
   Asp Gln Val Asp Val Lys Asp Cys Ala Asn His Glu Ile Lys Lys Val
   1               5                   10                  15
   Leu Val Pro Gly Cys His Gly Ser Glu Pro Cys Ile Ile His Arg Gly
               20                  25                  30
   Lys Pro Phe Gln Leu Glu Ala Val Phe Glu Ala Asn Gln Asn Thr Lys
           35                  40                  45
   Thr Ala Lys Ile Glu Ile Lys Ala Ser Ile Asp Gly Leu Glu Val Asp
       50                  55                  60
   Val Pro Gly Ile Asp Pro Asn Ala
   65                  70
<210> 17
   <211> 73
   <212> PRT
   <213> Dermatophagoides pteronyssinus
<400> 17
   Ser Ile Asp Gly Leu Glu Val Asp Val Pro Gly Ile Asp Pro Asn Ala
   1               5                   10                  15
   Cys His Tyr Met Lys Cys Pro Leu Val Lys Gly Gln Gln Tyr Asp Ile
               20                  25                  30
   Lys Tyr Thr Trp Asn Val Pro Lys Ile Ala Pro Lys Ser Glu Asn Val
           35                  40                  45
   Val Val Thr Val Lys Val Met Gly Asp Asp Gly Val Leu Ala Cys Ala
       50                  55                  60
   Ile Ala Thr His Ala Lys Ile Arg Asp
   65                  70
<210> 18
   <211> 25
   <212> PRT
   <213> Dermatophagoides pteronyssinus
<400> 18
   Asp Gln Val Asp Val Lys Asp Cys Ala Asn His Glu Ile Lys Lys Val
   1               5                   10                  15
   Leu Val Pro Gly Cys His Gly Ser Glu
               20                  25
<210> 19
   <211> 56
   <212> PRT
   <213> Dermatophagoides pteronyssinus
<400> 19
   His Gly Ser Glu Pro Cys Ile Ile His Arg Gly Lys Pro Phe Gln Leu
   1               5                   10                  15
   Glu Ala Leu Phe Glu Ala Asn Gln Asn Ser Lys Thr Ala Lys Ile Glu
               20                  25                  30
   Ile Lys Ala Ser Ile Asp Gly Leu Glu Val Asp Val Pro Gly Ile Asp
           35                  40                  45
   Pro Asn Ala Cys His Tyr Met Lys
       50                  55
<210> 20
   <211> 56
   <212> PRT
   <213> Dermatophagoides pteronyssinus
<400> 20
   His Tyr Met Lys Cys Pro Leu Val Lys Gly Gln Gln Tyr Asp Ile Lys
   1               5                   10                  15
   Tyr Thr Trp Asn Val Pro Lys Ile Ala Pro Lys Ser Glu Asn Val Val
               20                  25                  30
   Val Thr Val Lys Val Leu Gly Asp Asn Gly Val Leu Ala Cys Ala Ile
           35                  40                  45
   Ala Thr His Ala Lys Ile Arg Asp
       50                  55
<210> 21
   <211> 31
   <212> PRT
   <213> Dermatophagoides pteronyssinus
<400> 21
   Asp Gln Val Asp Val Lys Asp Cys Ala Asn His Glu Ile Lys Lys Val
   1               5                   10                  15
   Leu Val Pro Gly Cys His Gly Ser Glu Pro Cys Ile Ile His Arg
               20                  25                  30
<210> 22
   <211> 75
   <212> PRT
   <213> Dermatophagoides pteronyssinus
<400> 22
   Ser Glu Pro Cys Ile Ile His Arg Gly Lys Pro Phe Gln Leu Glu Ala
   1               5                   10                  15
   Leu Phe Glu Ala Asn Gln Asn Ser Lys Thr Ala Lys Ile Glu Ile Lys
               20                  25                  30
   Ala Ser Ile Asp Gly Leu Glu Val Asp Val Pro Gly Ile Asp Pro Asn
           35                  40                  45
   Ala Cys His Tyr Met Lys Cys Pro Leu Val Lys Gly Gln Gln Tyr Asp
       50                  55                  60
   Ile Lys Tyr Thr Trp Asn Val Pro Lys Ile Ala
   65                  70                  75
<210> 23
   <211> 41
   <212> PRT
   <213> Dermatophagoides pteronyssinus
<400> 23
   Lys Tyr Thr Trp Asn Val Pro Lys Ile Ala Pro Lys Ser Glu Asn Val
   1               5                   10                  15
   Val Val Thr Val Lys Val Leu Gly Asp Asn Gly Val Leu Ala Cys Ala
               20                  25                  30
   Ile Ala Thr His Ala Lys Ile Arg Asp
           35                  40
<210> 24
   <211> 57
   <212> PRT
   <213> Dermatophagoides pteronyssinus
<400> 24
   Ser Ile Asp Gly Leu Glu Val Asp Val Pro Gly Ile Asp Pro Asn Ala
   1               5                   10                  15
   Cys His Tyr Met Lys Cys Pro Leu Val Lys Gly Gln Gln Tyr Asp Ile
               20                  25                  30
   Lys Tyr Thr Trp Asn Val Pro Lys Ile Ala Pro Lys Ser Glu Asn Val
           35                  40                  45
   Val Val Thr Val Lys Val Leu Gly Asp
       50                  55
<210> 25
   <211> 21
   <212> PRT
   <213> Dermatophagoides pteronyssinus
<400> 25
   Lys Val Leu Gly Asp Asn Gly Val Leu Ala Cys Ala Ile Ala Thr His
   1               5                   10                  15
   Ala Lys Ile Arg Asp
               20
<210> 26
   <211> 32
   <212> PRT
   <213> Dermatophagoides pteronyssinus
<400> 26
   Ala Arg Glu Gln Ser Cys Arg Arg Pro Asn Ala Gln Arg Phe Gly Ile
   1               5                   10                  15
   Ser Asn Tyr Cys Gln Ile Tyr Pro Pro Asn Val Asn Lys Ile Arg Glu
               20                  25                  30
<210> 27
   <211> 320
   <212> PRT
   <213> Dermatophagoides pteronyssinus
<400> 27
   Met Lys Ile Val Leu Ala Ile Ala Ser Leu Leu Ala Leu Ser Ala Val
   1               5                   10                  15
   Tyr Ala Arg Pro Ser Ser Ile Lys Thr Phe Glu Glu Tyr Lys Lys Ala
               20                  25                  30
   Phe Asn Lys Ser Tyr Ala Thr Phe Glu Asp Glu Glu Ala Ala Arg Lys
           35                  40                  45
   Asn Phe Leu Glu Ser Val Lys Tyr Val Gln Ser Asn Gly Gly Ala Ile
       50                  55                  60
   Asn His Leu Ser Asp Leu Ser Leu Asp Glu Phe Lys Asn Arg Phe Leu
   65 70 75 80
   Met Ser Ala Glu Ala Phe Glu His Leu Lys Thr Gln Phe Asp Leu Asn
   85 90 95
   Ala Glu Thr Asn Ala Cys Ser Ile Asn Gly Asn Ala Pro Ala Glu Ile
   100 105 110
   Asp Leu Arg Gln Met Arg Thr Val Thr Pro Ile Arg Met Gln Gly Gly
   115 120 125
   Cys Gly Ser Cys Trp Ala Phe Ser Gly Val Ala Ala Thr Glu Ser Ala
   130 135 140
   Tyr Leu Ala Tyr Arg Asn Gln Ser Leu Asp Leu Ala Glu Gln Glu Leu
   145 150 155 160
   Val Asp Cys Ala Ser Gln His Gly Cys His Gly Asp Thr Ile Pro Arg
   165 170 175
   Gly Ile Glu Tyr Ile Gln His Asn Gly Val Val Gln Glu Ser Tyr Tyr
   180 185 190
   Arg Tyr Val Ala Arg Glu Gln Ser Cys Arg Arg Pro Asn Ala Gln Arg
   195 200 205
   Phe Gly Ile Ser Asn Tyr Cys Gln Ile Tyr Pro Pro Asn Val Asn Lys
   210 215 220
   Ile Arg Glu Ala Leu Ala Gln Thr His Ser Ala Ile Ala Val Ile Ile
   225 230 235 240
   Gly Ile Lys Asp Leu Asp Ala Phe Arg His Tyr Asp Gly Arg Thr Ile
   245 250 255
   Ile Gln Arg Asp Asn Gly Tyr Gln Pro Asn Tyr His Ala Val Asn Ile
   260 265 270
   Val Gly Tyr Ser Asn Ala Gln Gly Val Asp Tyr Trp Ile Val Arg Asn
   275 280 285
   Ser Trp Asp Thr Asn Trp Gly Asp Asn Gly Tyr Gly Tyr Phe Ala Ala
   290 295 300
   Asn Ile Asp Leu Met Met Ile Glu Glu Tyr Pro Tyr Val Val Ile Leu
   305 310 315 320
<210> 28
   <211> 129
   <212> PRT
   <213> Dermatophagoides pteronyssinus
<400> 28
   Asp Gln Val Asp Val Lys Asp Cys Ala Asn His Glu Ile Lys Lys Val
   1               5                   10                  15
   Leu Val Pro Gly Cys His Gly Ser Glu Pro Cys Ile Ile His Arg Gly
               20                  25                  30
   Lys Pro Phe Gln Leu Glu Ala Leu Phe Glu Ala Asn Gln Asn Ser Lys
           35                  40                  45
   Thr Ala Lys Ile Glu Ile Lys Ala Ser Ile Asp Gly Leu Glu Val Asp
       50                  55                  60
   Val Pro Gly Ile Asp Pro Asn Ala Cys His Tyr Met Lys Cys Pro Leu
   65                  70                  75                  80
   Val Lys Gly Gln Gln Tyr Asp Ile Lys Tyr Thr Trp Asn Val Pro Lys
                   85                  90                  95
   Ile Ala Pro Lys Ser Glu Asn Val Val Val Thr Val Lys Val Leu Gly
               100                 105                 110
   Asp Asn Gly Val Leu Ala Cys Ala Ile Ala Thr His Ala Lys Ile Arg
           115                 120                 125
   Asp
<210> 29
   <211> 146
   <212> PRT
   <213> Dermatophagoides pteronyssinus
<400> 29
   Met Met Tyr Lys Ile Leu Cys Leu Ser Leu Leu Val Ala Ala Val Ala
   1               5                   10                  15
   Arg Asp Gln Val Asp Val Lys Asp Cys Ala Asn His Glu Ile Lys Lys
               20                  25                  30
   Val Leu Val Pro Gly Cys His Gly Ser Glu Pro Cys Ile Ile His Arg
           35                  40                  45
   Gly Lys Pro Phe Gln Leu Glu Ala Val Phe Glu Ala Asn Gln Asn Thr
       50                  55                  60
   Lys Thr Ala Lys Ile Glu Ile Lys Ala Ser Ile Asp Gly Leu Glu Val
   65                  70                  75                  80
   Asp Val Pro Gly Ile Asp Pro Asn Ala Cys His Tyr Met Lys Cys Pro
                   85                  90                  95
   Leu Val Lys Gly Gln Gln Tyr Asp Ile Lys Tyr Thr Trp Asn Val Pro
               100                 105                 110
   Lys Ile Ala Pro Lys Ser Glu Asn Val Val Val Thr Val Lys Val Met
           115                 120                 125
   Gly Asp Asp Gly Val Leu Ala Cys Ala Ile Ala Thr His Ala Lys Ile
       130                 135                 140
   Arg Asp
   145

## Description

### FIELD OF THE INVENTION

The present invention relates to compositions and combinations of contiguous overlapping peptides (COPs) derived from the Der p 1 and Der p 2 house dust mite major allergens and the use of such compositions and combinations in medicine. The compositions and combinations and methods of treatment of the invention are contemplated to be useful in treating house dust mite allergy and widely accelerating its treatment.

### BACKGROUND OF THE INVENTION

IgE·mediated allergic disease appears to be very common particularly in industrialized countries where up to one quarter of the population is affected by allergic rhinitis [1]. Furthermore people suffering from allergic rhinitis show a lower quality of life than healthy ones [2], with only a few going into remission spontaneously.

House Dust Mites allergy is widely distributed worldwide and about 50% of the allergic population in the US and Europe suffers from allergy to house dust mites for review see [3]. House dust mites (HDM) belong predominantly to two species *Dermatophagoides pteronyssinus*, and *Dermatophagoides farinae*. Treatment against HDM allergy can be based on the major allergens of one of the two species since the major HDM allergens of *D*. *pteronyssinus* and *D. farinae*, Der p 1/ Der f 1 and Der p 2/ Der f 2 share over 80% sequence identity. Der p 1 shares 83% sequence identity with Der f 1 leading to highly cross-reactive human IgE antibody and T cell responses between these two species. In particular, pre-incubation of allergic patients' serum with Der f 1 has been found to prevent binding of Der p 1 to HDM IgE antibodies, even though different binding affinities were reported. Der p 2and Der f 2 (88% sequence identity) have been found to prevent binding of their counterparts very effectively. T cell proliferation was also found to be equally induced by *D. pteronyssinus* and *D. farinae* peptides. Allergens from the two species are not fully interchangeable however, since fewer peptides from Der f 1 than from Der p 1 were able to stimulate T cell proliferation for example.

Possible interchangeable use of allergens from different species has been indicated by immunotherapy results of a study conducted with *D. pteronyssinus* in Italian regions where the sensitizing species is *D. farinae* which did not differ markedly from those conducted in England with *D. pteronyssinus* for *D. pteronyssinus* sensitization or those conducted in South Korea with *D. farinae* for *D. farinae.* The results in Italy also did not differ from those conducted with a mixture of extracts as reviewed by Thomas [3]. Thus major allergens from *D. pteronyssinus* can be considered as the basis for an efficacious product at least against both *D. pteronyssinus*, *D. farinae* and possibly other house dust mite species.

Multiple allergen sequences have been described in HDM allergy, as found in the NCBI Nucleotide and protein databases. The allergens can be allocated to at least 7 groups related in sequence to known proteins, namely:
Der p 1, Cystein protease (peptidase C1A family)
Der p 2, MD2 like lipid binding protein, TLR 4 binding
Der p 3, trypsin-like serine protease
Der p 4, alpha-amylase
Der p 5, Blo t 5 (*Blomia tropicalis*, a storage mite)
Der p 6, chymotrypsin like (S1 peptidase domain)
Der p 7, a secreted glycoprotein

In Europe, 97% of the subjects allergic to HDM can be diagnosed using a mix of Der p 1 and Der p 2, whereas 50% of the patients react also to additional allergens of the Der p family [4]. In Brazil, about 87% of HDM prick positive patients with allergic rhinitis, were found to contain IgE recognizing Der p 1, Der p 2 or both allergens [5]. A study in Australia [6] shows that about 50% of anti HDM IgE antibodies bind to Der p 1 and Der p 2 and a further 30% was equally contributed by Der p 4, 5 and 7. Thus reactivity to Der p 1 and Der p 2 clearly dominates in the allergic population, indicating that a product based on Der p 1 and Der p 2 allergens may treat a vast majority of patients.
Der p 1 sequences and IgE epitopes
Der p 1 variants have been described indicating some polymorphism [7]. A BLAST search using the Swiss-Prot sequence P08176.2, revealed a number of protein sequences within the NCBI databases. Most variations were found in Der f 1 sequences and were located to 5 predominant positions, namely 123, 132, 150, 152 and 204 (coordinates referred to Swiss-Prot P08176.2). Polymorphism was also described using RT-PCR on a panel of sequences of the groups 1 and 2 allergens from both *D. pteronyssinus* and *D. farinae* isolated from homes in Bangkok [8]. Taken together these results indicate that the most predominant Der p 1 is the sequence referenced in Swiss-Prot P08176.2 , also identical to Der p 1.0105 [8].

The Der p 1 protein is a cysteine protease (Peptidase of the C1 superfamily) of 320 amino acids. The first 18 amino acids have properties of a signal peptide, whereas the next amino acids from R19 to E98 are present in the pro-protein and do contain a protease inhibitor domain 129. Mature Der p 1 is composed of 222 amino acids (T 99 to L320) and three crystal structures had been determined (PDB 1XKG, 2AS8 and 3F5V).

When searching the Immune Epitope Database (IEDB) for Der p 1, 35 epitopes were found. 24 B cell epitopes were described which were determined either by antigen competition, Western blotting, ELISA, Ig histamine release, or radio-immuno assay [9]; [10]; [11]; [12]. Combining these potential epitopes allowed delineating four main regions with boundaries located at N150 to H170; V187 to R202; C215 to Q231; Y263 to Y299.

IgE epitopes are mostly conformational and thus difficult to map. Monoclonal antibodies raised against either Der p 1 or Der f 1 are mostly species-specific. However, antibody 4C1 against Der f 1 binds also Der p 1 and the epitopes for the monoclonal antibody (mAb) and human IgE antibodies were found to overlap as determined by crystal structures of the complex [13]. Co-crystallization of 4C1 with Der p 1 and Der f 1 indicated residues D113, R115, Q116, R118, R154, 1156, Q279, Y283, D296, Y299, Y301 possibly involved in IgE binding.

mAb W108 not only inhibited the binding of Der p 1 with IgE antibodies but also its cysteine proteinase activity [14]. Three peptides were identified by LC-MS after protease digestion of the W108/Der p 1 complex (aa 209-224; 227-243 and 260-287) which did not overlap with two peptide segments of Der p 1 found to bind most directly to mAb W108 (aa 151-197 and 286-320). These results demonstrate the complexity of mapping IgE epitopes even when using monoclonal antibodies.

Summarizing data from the literature allowed to identify four regions of Der p 1 with potential for binding to IgE, namely N150-H170, V187-R202, C215-Q231 and G274-D291. The reported experiments show that splitting these four domains of Der p 1 was not sufficient, since an additional region between R149 to R254 had to be interrupted to remove residual IgE binding.

Der p 2 sequences and IgE epitopes

Der p 2 seems to be very polymorphic with 15 described variants, including the best characterized versions Der p2.0101, 0103, 0104, 0107 and 0108 [15]. According to [8], Der p 2 showed frequent variations with clusters of amino acid substitutions, but the canonical Der p 2.0101 was not found in any of the 17 sequences. Der f 2 showed variants with clusters of substitutions similar to Der p 2 but in different amino acid positions and without any structural concordance.

According to [16], both variants, Der p 2.0101 and Der p 2.0104 were the most active for T cell stimulation, whereas other less common variants, namely 0107 and 0108 showed consistent differences demonstrating that changes in the sequence could change the cytokine response. According to [15], Der p 2 isoforms 0103 and 0104 seem to be bound with a higher affinity to a series of recombinant IgEs obtained by phage display. These two isoforms combined with the rIgEs showed accordingly a better ability to trigger degranulation in a reconstitution assay. However, all Der p 2 isoforms were able to trigger degranulation in presence of polyclonal sera from allergic patients. Taking into consideration the above results, in particular T cell response and high affinity for selected rIgE clones, the Der p 2.0104 variant (GenBank AFJ68067.1 was chosen for product definition.

Different techniques have been used in an attempt to determine IgE epitopes, including Hydrogen Exchange Nuclear Magnetic Resonance [17] and Mimotopes [18]. Three major regions seem to be involved located on the surface of the molecule, namely N71-C78, V94-K100 and L111-G115 (numbering according to GenBank AFJ68067.1 sequence). The last two regions include residues identified in the IEDB database as being part of possible IgE epitopes [7]; However, no definitive epitope mapping was proposed due to the 3D binding specificity of IgEs.

### Hypoallergenic allergy vaccines

The only treatment directed to the cause of IgE-mediated allergy is specific immunotherapy (SIT). The treatment consists in injecting increasing doses of allergens for extended periods of time (three to five years) to induce tolerance in the allergic patient. Several studies showed the benefit of this therapy on the allergic response, in particular upon long-term treatment [19], [20]. However, a number of side effects were observed particularly during ultra-rush therapies, where up to 30% of the patients have to be treated for allergic symptoms during the course of therapy [21]. There is thus a strong medical need for an alternative to SIT in the form of a shorter treatment with acceptable safety.

Different approaches have been tested to improve the safety and efficacy of SIT. Formulations or existing extracts have been improved by adding adjuvants, like MPL (Allergy Therapeutics) [22], DNA sequences [23], or bacteriophage combined with CpG [24] which increase the TH1 immune response, thus allowing possible reductions in the amount of allergen extract. Defined allergens were used instead of whole extracts. In the case of birch pollen, a clinical trial with recombinant Bet v 1 has shown efficacy equivalent to whole birch pollen extract [25].

To diminish the occurrence of allergic symptoms resulting from treatment, different groups explored the use of products with hypoallergenic potential, namely showing reduced IgE binding. Expression of Der p 1 in E. coli resulted simply in aggregated proteins which showed reduced IgE binding [26] and was proposed as possible hypoallergenic vaccine. Expression of pro-Der p 1 in P. pastoris resulted in a stable hypoallergenic pro-enzyme also with potential for use in allergen-specific immunotherapy [27]. Combination of allergens, namely Der p 1 / Der p 2 hybrid proteins were engineered by PCR [28] or hybrid proteins were reassembled with Der p 1 and Der p 2 fragments [29] and expressed in E. coli. Lowered IgE reactivity was shown in both cases while preserving immunogenicity. A potential DNA vaccine candidate with optimized codons has also been constructed [30], [31]. However, none of these approaches have been tested in human yet.

A further approach consisted in providing peptides encompassing a restricted number of T-cell epitopes which were used for allergen immunotherapy of cat dander with limited efficacy [32]. The US patent US 6,268,491 discloses, for example, peptides of a protein allergen selected from the allergens Der p I, Der p II, Der f I, or Der f II, said peptide comprising at least one T-cell epitope. However, allergens harbor a great variety of T cell epitopes partly dependent on the HLA type of the patient. For example, T cell epitopes were found scattered throughout the Bet v 1 sequence, except for a short region [33]. Thus an efficient immunotherapy product should preferably contain the complete sequence of the allergen rather than selected T-cell epitopes.

The use of fragments of allergens remains attractive, based on the evidence that human IgE recognize mainly non-contiguous epitopes which may be separated by fragmentation of the allergen. Two contiguous fragments of Bet v 1 or trimeric forms of Bet v 1 were tested in a phase I study in human and showed a trend towards improvement of wellbeing but provided no significant improvement in symptom medication scores [34]. In that study, however, a number of adverse events were observed, the majority of which occurred hours after the injections [35]. Three fragments of the major allergen of bee venom, namely phospholipase A2, were also tested in human, showing an excellent safety due to lowered IgE binding while eliciting elevated levels of IgG4 and IL-10 [36]. A method was devised to select contiguous overlapping peptides (COPs) for treatment of allergy which together form the entire amino acid sequence of an allergen, thus providing all possible T cell epitopes of the allergen, while having lowered IgE binding (US Patent 7,923,209). Such selected fragments show a reduced ability to reform the original tertiary structure of the allergen, if any, resulting in a reduced ability to bind IgE and therefore to elicit allergic reactions in humans.

### SUMMARY OF THE INVENTION

The invention is as defined in the claims.

According to one aspect, the present invention provides compositions and combinations as defined in the claims comprising contiguous overlapping peptides (COPs) as defined in the claims for the treatment of house dust mites' allergies. Specifically, COPS disclosed herein are obtained from the sequence of the two major allergens of house dust mites Der p 1 and Der p 2 which include the complete sequence of these allergens and thus provide all potential T cell epitopes, but are devoid of the three dimensional structure of the original allergen.

The COPS may be used in methods of specific immunotherapy against house mite dust allergies and may be administered to mice and other mammals sensitized with Der p1 or Der p 2 or a mix of these two allergens without eliciting anaphylactic shock. More specifically, the specification discloses a specific immunotherapy (SIT) method able to reduce allergic symptoms after a few administrations over a short period of time. This therapy consists of repeatedly administering specific COPs to humans suffering from allergy to house dust mites. Administration may be done by systemic, transdermal, intradermal subcutaneous, or by oral routes, or mucosal routes including sublingual and intestinal routes. Administration may in some embodiments be repeated five times over two month compared to 3 to 5 years for current SIT. Administered amount of active product (COPs) may reach a cumulated value equivalent in molar amount to the amount of Der p 1 and Der p 2 administered over three year of SIT treatment.

Specifically the invention refers to a composition comprising six contiguous overlapping peptides, wherein
- the first peptide consists of the sequence from amino acid T99 to amino acid G168 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acid A164 to amino acid S200 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid R193 to amino acid E227 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the fourth peptide consists of the sequence from amino acidP220 to amino acid R254 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the fifth peptide consists of the sequence from amino acid R249 to amino acid D282 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained, and
- the sixth peptide consists of the sequence from amino acid A278 to amino acid L320 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained.

The invention further refers to a composition comprising four contiguous overlapping peptides, wherein
- the first peptide consists of the sequence from amino acid D1 to amino acid E25 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acid H22 to amino acid K77 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid S57 to amino acid D113 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained, and- the fourth peptide consists of the sequence from amino acid K109 to amino acid D129 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained.

Specifically the invention provides compositions comprising a plurality of contiguous overlapping peptide fragments (COPs) wherein the reactivity of said COPs to IgE antibodies of subjects who are allergic to house dust mites is substantially reduced or eliminated while the reactivity with the T lymphocytes from subjects who are allergic to house dust mites is retained. As used herein the statement that reactivity to IgE antibodies is eliminated is understood by those of skill in the immunology art that such reactivity is reduced by three or four or more logs to a level at which it is clinically irrelevant or by which it is undetectable by ordinary measurement techniques.

The invention further refers to a combination of allergens for use in a method of specific immunotherapy against house dust mites allergies in a patient, wherein said combination of allergens comprises ten peptides, wherein
- the first peptide consists of the sequence from amino acid T99 to amino acid G168 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acid A164 to amino acid S200 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid R193 to amino acid E227 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the fourth peptide consists of the sequence from amino acid P220 to amino acid R254 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the fifth peptide consists of the sequence from amino acid R249 to amino acid D282 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the sixth peptide consists of the sequence from amino acid A278 to amino acid L3220 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the seventh peptide consists of the sequence from amino acid D1 to amino acid E25 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained,
- the eighth peptide consists of the sequence from amino acid H22 to amino acid K77 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained,
- the ninth peptide consists of the sequence from amino acid S57 to amino acid D113 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained, and
- the tenth peptide consists of the sequence from amino acid K109 to amino acid D129 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained,
and wherein said combination of ten allergens is administered to a patient in need thereof.

Said combination of COPs including both the complete sequences of Der p 1 (Swiss-Prot P08176.2) and the complete sequence of Der p 2 (GenBank AFJ68067.1), said Der p 1 sequences are obtained by the addition of a first peptide starting at amino acid T99 and ending at G168 (SEQ ID NO: 8), a second peptide starting at amino acid A164 and ending at S200 (SEQ ID NO: 12), a third peptide starting at amino acids R193 and ending at E227 (SEQ ID NO: 13), a fourth peptide starting at amino acids P220 and ending at R254 (SEQ ID NO: 14), a fifth peptide starting at amino acids R249 and ending at D282 (SEQ ID NO: 15), a sixth peptide starting at amino acids A278 and ending at L320 (SEQ ID NO: 11), said Der p 2 sequences are obtained by addition of a first peptide starting at amino acids D1 and ending at E25 (SEQ ID NO: 18), a second peptide starting at amino acids H22 and ending at K77 (SEQ ID NO: 19), a third peptide starting at amino acids S57 and ending at D113 (SEQ ID NO: 24), and a fourth peptide starting at amino acids K109 and ending at D129 (SEQ ID NO: 25).

While the sequences above represent the peptides for overlapping slightly shorter and slightly longer versions of each of those first through sixth Der p 1 peptides and first through fourth Der p 2 peptides are herein disclosed which are each 1-3 or 1-5 or 1-10 or 1-15 amino acids truncated or elongated from the peptides of SEQ ID NOS: 8, 11-15, 18, 19, 24 and 25 such that IgE reactive three dimensional epitopes are not recreated and such that the reactivity of the peptide to IgE antibodies of subjects who are allergic to Der p 1 and/or Der p 2 is eliminated and whereby sets of peptides which overlap each other by one or more amino acids are produced such that reactivity with T lymphocytes from subjects who are allergic to house dust mites is maintained. The truncated or elongated COPs will be equivalent functionally to the peptides of SEQ ID NOS: 8, 11-15, 18, 19, 24 and 25 provided that they do not lead to restored IgE binding. It is herein disclosed that there exists some sequence variability in the major dust mite allergens Der p 1 and Der p 2. Thus one sequence of Der p 2 GenBank sequence AFJ68067.1 (SEQ ID NO: 28 differs by four amino acid residues from the mature protein sequence variant in Swiss Prot P49278.1 (SEQ ID NO: 29). Specifically referring to SEQ 28 the sequences vary by the substitution of a hydrophobic valine (V) for a hydrophilic lysine (L) at AA 40, the substitution of a nucleophilic serine (S) for a nucleophilic threonine (T) at AA 47, the substitution of a hydrophobic lysine (L) for a hydrophobic methionine (M) at AA 70 and the substitution of an amide asparagine (N) for an acidic residue aspartic acid (D) at AA 114. Those of skill in the art would be capable of selecting from the sequences of different allergen isotypes as well as substituting amino acid residues having similar properties to obtain peptides useful for carrying out the specific immunotherapy methods of the specification. Accordingly the specification discloses COPs having 70%, 80%, 85%, 90% or 95% sequence identity to the peptides of SEQ ID NOS: 8, 11-15, 18, 19, 24 and 25 and the peptides of SEQ ID NOS: 8 and 11-15 which are elongated or truncated by 1-3, 1-5, 1-10 or 1-15 amino acids along SEQ ID NO: 26 and SEQ ID NOS: 18, 19, 24 and 25 along SEQ ID NO: 27 and wherein the reactivity of such peptides to IgE antibodies of subjects allergic to house dust mites is eliminated while reactivity with the T lymphocytes from subjects who are allergic to house dust mites is retained.

As disclosed herein, the first and second peptides of Der p 1 overlap each other by 1 to 5 amino acids. As disclosed herein the second and third peptides of Der p 1 overlap each other by 1 to 8 amino acids. As disclosed herein the third and fourth peptides of Der p 1 overlap each other by 1 to 8 amino acids. As disclosed herein the fourth and fifth peptides of Der p 1 overlap each other by 1 to 6 amino acids. As disclosed herein the fifth and sixth peptides of Der p 1 overlap each other by 1 to 5 amino acids. As disclosed herein, the first and second peptides of Der p 2 overlap each other by 1 to 4 amino acids. As disclosed herein, the second and third peptides of Der p 2 overlap each other by 1 to 21 amino acids. As disclosed herein, the third and fourth peptides of Der p 2 overlap each other by 1 to 5 amino acids. According to one aspect of the invention, the compositions comprise the combination of the peptide having SEQ ID NO: 8, the peptide having SEQ ID NO: 12, the peptide having SEQ ID NO: 13, the peptide having SEQ ID NO: 14, the peptide having SEQ ID NO: 15, the peptide having SEQ ID NO: 11, the peptide having SEQ ID NO: 18, the peptide having SEQ ID NO: 19, the peptide having SEQ ID NO: 24 and the peptide having SEQ ID NO: 25.

Preferred COP compositions include those wherein the peptides are soluble in aqueous buffers compatible with application routes. As herein disclosed, the set of peptides AllerDM1.5, AllerDM1.52, AllerDM1.53, AllerDM1.54 and AllerDM1.5a are preferred to AllerDM1.2 due to their solubility in water. As herein disclosed, AllerDM1.7 is preferred to AllerDM1.3 and AllerDM1.72 as no polar aprotic solvent is needed for solubilization.

Such peptides can be obtained by any of a variety of methods including by chemical synthesis or by recombinant means.

The COPs and peptides as herein disclosed can be provided in dry powdered form but can also be provided in combination with an acceptable carrier or diluent. In addition, the compositions can further comprise an adjuvant with a preferred adjuvant being aluminum hydroxide. As such the compositions can be characterized as and used as a vaccine composition.

The COPs and peptides as herein disclosed can be provided together with denaturing and or chaotropic agents, comprising guanidinium chloride. Such agents function by preventing 3-dimensional structure formation in solution contribute to lower IgE binding.

Also herein disclosed are methods of specific immunotherapy (SIT) against house dust mite allergies comprising administering to a patient in need thereof one or more allergens selected from the group consisting of a combination of COPs including both the complete sequences of Der p 1 (Swiss-Prot P08176.2) and the complete sequence of Der p 2 (GenBank AFJ68067.1), said Der p 1 sequences are obtained by the addition of a first peptide starting at amino acid T99 and ending at G168 (SEQ ID NO: 8), a second peptide starting at amino acid A164 and ending at S200 (SEQ ID NO: 12), a third peptide starting at amino acids R193 and ending at E227 (SEQ ID NO: 13), a fourth peptide starting at amino acids P220 and ending at R254 (SEQ ID NO: 14), a fifth peptide starting at amino acids R249 and ending at D282 (SEQ ID NO: 15), a sixth peptide starting at amino acids A278 and ending at L320 (SEQ ID NO: 11), said Der p 2 sequences are obtained by addition of a first peptide starting at amino acids D1 and ending at E25 (SEQ ID NO: 18), a second peptide starting at amino acids H22 and ending at K77 (SEQ ID NO: 19), a third peptide starting at amino acids S57 and ending at D113 (SEQ ID NO: 24), and a fourth peptide starting at amino acids K109 and ending at D129 (SEQ ID NO: 25).

Such methods can be carried out in which the peptides are administered using intradermal injection, subcutaneous injection, intramuscular injection, intravenous injection, transdermal, intranasal, oral, sublingual, intraocular, or intrathecal techniques.

According to one such method, a patient is treated with the combination of COPs including both the complete sequences of Der p 1 (Swiss-Prot P08176.2) and the complete sequence of Der p 2 (GenBank AFJ68067.1), said Der p 1 sequences are obtained by the addition of a first peptide starting at amino acid T99 and ending at G168 (SEQ ID NO: 8), a second peptide starting at amino acid A164 and ending at S200 (SEQ ID NO: 12), a third peptide starting at amino acids R193 and ending at E227 (SEQ ID NO: 13), a fourth peptide starting at amino acids P220 and ending at R254 (SEQ ID NO: 14), a fifth peptide starting at amino acids R249 and ending at D282 (SEQ ID NO: 15), a sixth peptide starting at amino acids A278 and ending at L320 (SEQ ID NO: 11), said Der p 2 sequences are obtained by addition of a first peptide starting at amino acids D1 and ending at E25 (SEQ ID NO: 18), a second peptide starting at amino acids H22 and ending at K77 (SEQ ID NO: 19), a third peptide starting at amino acids S57 and ending at D113 (SEQ ID NO: 24), and a fourth peptide starting at amino acids K109 and ending at D129 (SEQ ID NO: 25). The specification further discloses a composition comprising a plurality of peptide fragments comprising a first polypeptide consisting of the sequence from amino acids 99-104 to amino acids 157-177 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a second peptide consisting of the sequence from amino acids 154-174 to amino acids 190-210 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a third peptide consisting of the sequence from amino acids 183-203 to amino acids 217-237 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a fourth peptide consisting of the sequence from amino acids 210-230 to amino acids 244-264 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a fifth peptide consisting of the sequence from amino acids 239-259 to amino acids 272-292 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a sixth peptide consisting of the sequence from amino acids 268-288 to amino acids 310-320 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained.

The specification further discloses compositions wherein the first and second peptides overlap each other by 1 to 5 amino acids; the second and third peptides overlap each other by 1 to 8 amino acids; the third and fourth peptides overlap each other by 1 to 8 amino acids; the fourth and fifth peptides overlap each other by 1 to 6 amino acids or the fifth and sixth peptides overlap each other by 1 to 5 amino acids.

The specification further discloses a composition comprising a plurality of peptide fragments comprising a first polypeptide consisting of the sequence from amino acids 1-15 to amino acids 15-35 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a second peptide consisting of the sequence from amino acids 12-32 to amino acids 67-87 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a third peptide consisting of the sequence from amino acids 47-67 to amino acids 103-123 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a fourth peptide consisting of the sequence from amino acids 99-119 to amino acids 119-129 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained.

The specification further discloses compositions wherein the first and second peptides overlap each other by 1 to 4 amino acids; the second and third peptides overlap each other by 1 to 21 amino acids or the third and fourth peptides overlap each other by 1 to 5 amino acids.

The specification further discloses a composition comprising a plurality of Der p1 contiguous overlapping peptide fragments comprising a first polypeptide consisting of the sequence from amino acids 99-104 to amino acids 157-177 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a second peptide consisting of the sequence from amino acids 154-174 to amino acids 190-210 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a third peptide consisting of the sequence from amino acids 183-203 to amino acids 217-237 of SEQ ID NO:27 wherein the reactivity of said peptides to IgE antibodies of subjects who are allergic to house dust mites is eliminated while the reactivity with the T lymphocytes from subjects who are allergic to house dust mites is retained, a fourth peptide consisting of the sequence from amino acids 210-230 to amino acids 244-264 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a fifth peptide consisting of the sequence from amino acids 239-259 to amino acids 272-292 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a sixth peptide consisting of the sequence from amino acids 268-288 to amino acids 310-320 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained; and a plurality of Der p2 contiguous overlapping peptide fragments comprising a first polypeptide consisting of the sequence from amino acids 1-15 to amino acids 15-35 of SEQ ID NO:28 wherein the reactivity of said peptides to IgE antibodies of subjects who are allergic to house dust mites is eliminated while the reactivity with the T lymphocytes from subjects who are allergic to house dust mites is retained, a second peptide consisting of the sequence from amino acids 12-32 to amino acids 67-87 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a third peptide consisting of the sequence from amino acids 47-67 to amino acids 103-123 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a fourth peptide consisting of the sequence from amino acids 99-119 to amino acids 119-129 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained.

Further disclosed is herein one method a patient in need of specific immunotherapy for dust mite allergy is treated with the combination of one or more Der p 1 contiguous overlapping peptides comprising a first peptide starting at amino acid T99 and ending at G 168 of a polypeptide having 90% sequence identity to (SEQ ID NO: 8), a second peptide starting at amino acid A164 and ending at S200 of a polypeptide having 90% sequence identity to (SEQ ID NO: 12), a third peptide starting at amino acids R193 and ending at E227 of a polypeptide having 90% sequence identity to (SEQ ID NO: 13), a fourth peptide starting at amino acids P220 and ending at R254 of a polypeptide having 90% sequence identity to (SEQ ID NO: 14), a fifth peptide starting at amino acids R249 and ending at D282 of a polypeptide having 90% sequence identity to (SEQ ID NO: 15), a sixth peptide starting at amino acids A278 and ending at L320 (SEQ ID NO: 11) which first through sixth peptides are optionally elongated or truncated by from 1-3, 1-5, 1-10 or 1-15 amino acids along SEQ ID NO: 27, and a plurality of Der p 2 contiguous overlapping peptides comprising a first peptide starting at amino acids D1 and ending at E25 of a polypeptide having 90% sequence identity to (SEQ ID NO: 18), a second peptide starting at amino acids H22 and ending at K77 of a polypeptide having 90% sequence identity to (SEQ ID NO: 19), a third peptide starting at amino acids S57 and ending at D113 of a polypeptide having 90% sequence identity to (SEQ ID NO: 24), and a fourth peptide starting at amino acids K109 and ending at D129 of a polypeptide having 90% sequence identity to (SEQ ID NO: 25) which first through fourth peptides are optionally elongated or truncated by from 1-3, 1-5, 1-10 or 1-15 amino acids along SEQ ID NO: 28 wherein the reactivity of said peptide to IgE antibodies of subjects who are allergic to house dust mites is eliminated while the reactivity with the T lymphocytes from subjects who are allergic to house dust mites is retained. According to preferred aspects of the invention the first and second peptides overlap each other by 1 to 5 amino acids. the first and second peptides overlap each other by 1 to 11 amino acids; the second and third peptides overlap each other by 1 to 8 amino acids; the third and fourth peptides overlap each other by 1 to 8 amino acids; the fourth and fifth peptides overlap each other by 1 to 6 amino acids; the fifth and sixth peptides overlap each other by 1 to 5 amino acids; the seventh and eighth peptides overlap each other by 1 to 4 amino acids; the eighth and ninth peptides overlap each other by 1 to 21 amino acids; and/or the ninth and tenth peptides overlap each other by 1 to 5 amino acids. As disclosed herein the patient in need thereof is treated with at least five or six or seven or eight or nine or ten of said allergens.

Peptides disclosed herein include a peptide comprising the sequence from amino acid T99 to G 168 of a polypeptide having 90% or 95% sequence identity to (SEQ ID NO: 8) which is optionally elongated or truncated by from 1-3, 1-5, 1-10 or 1-15 amino acids along SEQ ID NO: 27 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 8.

Peptides disclosed herein include a peptide comprising the sequence from amino acid A164 and ending at S200 of a polypeptide having 90% or 95% sequence identity to (SEQ ID NO: 12) which is optionally elongated or truncated by from 1-3, 1-5, 1-10 or 1-15 amino acids along SEQ ID NO: 27 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 12.

Peptides disclosed herein include a peptide comprising the sequence from amino acid R193 and ending at E227 of a polypeptide having 90% or 95% sequence identity to (SEQ ID NO: 13) which is optionally elongated or truncated by from 1-3, 1-5, 1-10 or 1-15 amino acids along SEQ ID NO: 27 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 13.

Peptides disclosed herein include a peptide comprising the sequence from amino acid P220 and ending at R254 of a polypeptide having 90% or 95% sequence identity to (SEQ ID NO: 14) which is optionally elongated or truncated by from 1-3, 1-5, 1-10 or 1-15 amino acids along SEQ ID NO: 27 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 14.

Peptides disclosed herein include a peptide comprising the sequence from amino acid R249 and ending at D282 of a polypeptide having 90% or 95% sequence identity to (SEQ ID NO: 15) which is optionally elongated or truncated by from 1-3, 1-5, 1-10 or 1-15 amino acids along SEQ ID NO: 27 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 15.

Peptides disclosed herein include a peptide comprising the sequence from amino acid A278 and ending at L320 of a polypeptide having 90% or 95% sequence identity to (SEQ ID NO: 11) which is optionally elongated or truncated by from 1-3, 1-5, 1-10 or 1-15 amino acids along SEQ ID NO: 27 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 11.

Peptides disclosed herein include a peptide comprising the sequence from amino acid D1 and ending at E25 of a polypeptide having 90% or 95% sequence identity to (SEQ ID NO: 18) which is optionally elongated or truncated by from 1-3, 1-5, 1-10 or 1-15 amino acids along SEQ ID NO: 28 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 18.

Peptides disclosed herein include a peptide comprising the sequence from amino acid D1 and ending at E25 of a polypeptide having 90% or 95% sequence identity to (SEQ ID NO: 18) which is optionally elongated or truncated by from 1-3, 1-5, 1-10 or 1-15 amino acids along SEQ ID NO: 28 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 18.

Peptides disclosed herein according to the invention include a peptide comprising the sequence from amino acid H22 and ending at K77 of a polypeptide having 90% or 95% sequence identity to (SEQ ID NO: 19) which is optionally elongated or truncated by from 1-3, 1-5, 1-10 or 1-15 amino acids along SEQ ID NO: 28 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 19.

Peptides disclosed herein include a peptide comprising the sequence from amino acid S57 and ending at D113 of a polypeptide having 90% or 95% sequence identity to (SEQ ID NO: 24) which is optionally elongated or truncated by from 1-3, 1-5, 1-10 or 1-15 amino acids along SEQ ID NO: 28 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 24.

Peptides disclosed herein include a peptide comprising the sequence from amino acid K109 and ending at D129 of a polypeptide having 90% or 95% sequence identity to (SEQ ID NO: 25) which is optionally elongated or truncated by from 1-3, 1-5, 1-10 or 1-15 amino acids along SEQ ID NO: 28 wherein the reactivity of said peptide to IgE antibodies of subjects who are allergic to house dust mites is eliminated while the reactivity with the T lymphocytes from subjects who are allergic to house dust mites is retained with a particularly preferred peptide having the sequence consisting of that of SEQ ID NO: 25.

The specification also discloses a method of specific immunotherapy against house dust mites allergies comprising administering to a patient in need thereof one or more allergens selected from the group consisting of a first polypeptide consisting of the sequence from amino acids 99-104 to amino acids 157-177 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a second peptide consisting of the sequence from amino acids 154-174 to amino acids 190-210 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a third peptide consisting of the sequence from amino acids 183-203 to amino acids 217-237 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a fourth peptide consisting of the sequence from amino acids 210-230 to amino acids 244-264 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a fifth peptide consisting of the sequence from amino acids 239-259 to amino acids 272-292 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a sixth peptide consisting of the sequence from amino acids 268-288 to amino acids 310-320 of a polypeptide having 90% sequence identity to SEQ ID NO:27 wherein the reactivity of said peptides to IgE antibodies of subjects who are allergic to house dust mites is eliminated while the reactivity with the T lymphocytes from subjects who are allergic to house dust mites is retained, a seventh polypeptide consisting of the sequence from amino acids 1-15 to amino acids 15-35 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, an eighth peptide consisting of the sequence from amino acids 12-32 to amino acids 67-87 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a ninth peptide consisting of the sequence from amino acids 47-67 to amino acids 93-123 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, and a tenth peptide consisting of the sequence from amino acids 99-119 to amino acids 119-129 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained.

Also disclosed is a composition for conducting specific immunotherapy comprising a plurality of contiguous overlapping peptide fragments which fragments together comprise the entire amino acid sequence of a polypeptide having 90% sequence identity to Der p 2 (SEQ ID NO: 28) comprising a first polypeptide consisting of the sequence from amino acid 1 to amino acids 30-82 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a second peptide consisting of the sequence from amino acids 12-32 to amino acids 67-87 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a third peptide consisting of the sequence from amino acids 47-67 to amino acids 103-123 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, and a fourth peptide consisting of the sequence from amino acids 89-109 to amino acid 129 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained.

Also disclosed is a composition for conducting specific immunotherapy comprising a plurality of contiguous overlapping peptide fragments which fragments together comprise the entire amino acid sequence of a polypeptide having 90% sequence identity to Der p 2 (SEQ ID NO: 28) comprising a first polypeptide consisting of the sequence from amino acid 1 to amino acids 62-82 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, a second peptide consisting of the sequence from amino acids 47-67 to amino acids 103-123 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained, and a third peptide consisting of the sequence from amino acids 89-119 to amino acid 129 of a polypeptide having 90% sequence identity to SEQ ID NO:28 wherein the reactivity of said peptides to IgE antibodies of subjects who are allergic to house dust mites is eliminated while the reactivity with the T lymphocytes from subjects who are allergic to house dust mites is retained. Particularly preferred is the composition wherein the second peptide starting at amino acid S57 and ending at D113 (SEQ ID NO: 24) is neither elongated nor truncated.

Further disclosed is a composition for conducting specific immunotherapy comprising a plurality of Der p 2 contiguous overlapping peptide fragments comprising a first peptide starting at amino acid D1 and ending at A72 of (SEQ ID NO: 16), a second peptide starting at amino acid S57 and ending at D113 (SEQ ID NO: 24), and a third peptide selected from the group consisting of the peptide starting at amino acid K89 and ending at D129 of (SEQ ID NO: 28) or the peptide starting at amino acid K109 and ending at D129 (SEQ ID NO: 25) wherein the peptides are optionally elongated or truncated by from 1-3, 1-5, 1-10 or 1-15 amino acids along SEQ ID NO: 28 and wherein the reactivity of said peptides to IgE antibodies of subjects who are allergic to house dust mites is eliminated while the reactivity with the T lymphocytes from subjects who are allergic to house dust mites is retained. Also disclosed is the composition wherein the second peptide starting at amino acid S57 and ending at D113 (SEQ ID NO: 24) is neither elongated nor truncated.

The compositions of the invention may be in dry powdered form and may further comprise a pharmaceutically acceptable carrier or diluent and/or an adjuvant with aluminium hydroxide being a particularly preferred adjuvant.

As disclosed herein, the peptides, combinations of peptides and compositions comprising the same may be administered in methods of specific immunotherapy against house dust mites allergies comprising administering to a patient in need thereof one or more allergens using intradermal injection, subcutaneous injection, intramuscular injection, intravenous injection, transdermal, intranasal, oral, sublingual, intraocular, or intrathecal techniques.

As disclosed herein it has been discovered that carrying out specific immunotherapy by administration of the individual peptides as disclosed herein and overlapping peptides as disclosed herein can be promoted by the co-administration of the peptides in the presence of a denaturing or chaotropic agent with guanidinium chloride being a particularly preferred agent.

### BRIEF DESCRIPTION OF THE DRAWINGS

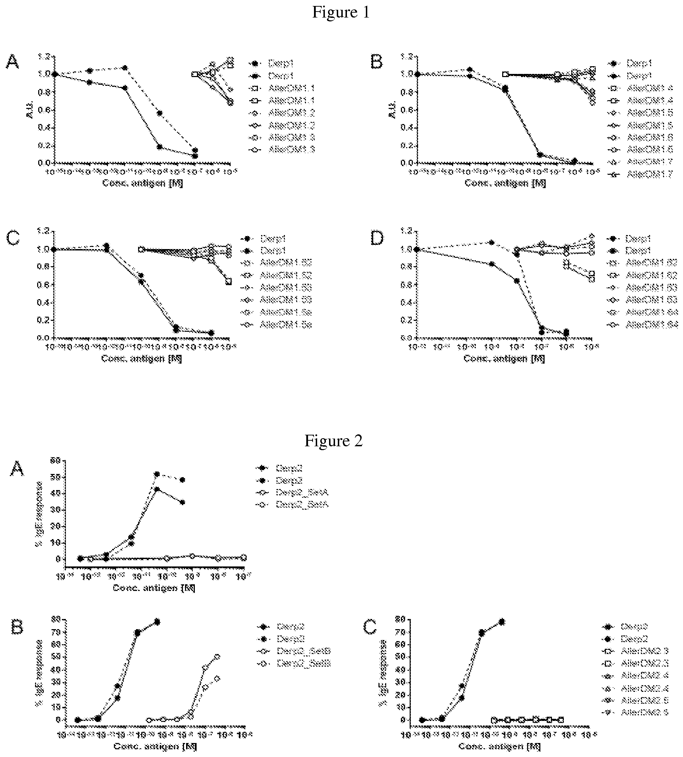

Fig. 1 depicts the competitive binding of selected COPs to IgE from two serums (continuous and dashed line) compared to Der p 1.Panel A: Der p1 (closed symbols) vs. single peptides AllerDM1.1, 1.2 and 1.3, (open symbols). Panel B Der p1 (closed symbols) vs. single peptides AllerDM1.4, 1.5 and 1.6 and 1.7 (open symbols). Panel C: Der p1 (closed symbols) vs. single peptides AllerDM1.52, 1.53 and 1.5a. Panel D: Der p1 (closed symbols) vs. single peptides AllerDM1.62, 1.63 and 1.64.
Fig. 2 depicts the ability of Der p 2 and selected COPs to induce degranulation of "humanized" RBL cells (obtained from Dr. Vogel, Paul-Ehrlich-Institute, Langen, Germany) pre-incubated with two different human serums. Panel A: Der p2 (closed symbols) vs. Derp2_SetA (open symbols). Panel B: Der p2 (closed symbols) vs. Derp2_SetB (open symbols). Panel C: Der p2 (closed symbols) vs. single peptides AllerDM2.3, 2.4 and 2.5.

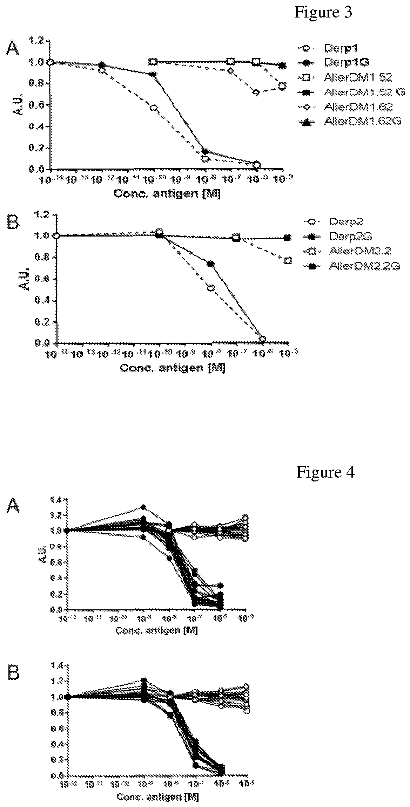

Fig. 3 depicts the reduction of IgE binding resulting from the use of guanidinium chloride in competition ELISA. Panel A: Der p 1, AllerDM1.52 and AllerDM1.62 were tested with (label G) or without 30 min pre-incubation with 1M guanidinium chloride. Panel B: Der p 2 and AllerDM2.2 were tested with or without 30 min pre-incubation with 1M guanidinium chloride.
Fig. 4 depicts the lack of IgE binding of the selected product AllerDM composed of an equimolar mix of 10 COPs. 21 serums of HDM allergic subjects from Europe and USA were tested in competition ELISA. Panel A: competition with Der p1 (closed symbols) vs. AllerDM (open symbols) for Der p 1 reactivity. Panel B: competition with Der p2 (full symbols) vs. AllerDM (open symbols) for Der p 2 reactivity.

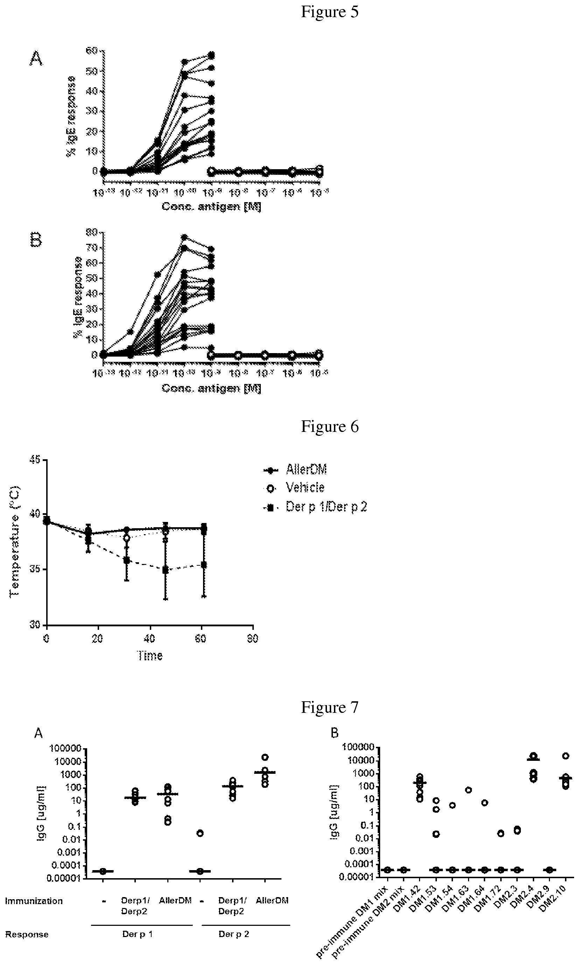

Fig. 5 depicts the inability of the selected product AllerDM composed of an equimolar mix of 10 COPs to induce degranulation of RBL cells loaded with 18 sera from HDM allergic patients. Panel A: Der p1 (full symbols) vs. AllerDM (open symbols) induced degranulation. Panel B: Der p2 (full symbols) vs. AllerDM (open symbols) induced degranulation.
Figure 6 depicts the anaphylactic reactions of Balb/c mice sensitized by repeated subcutaneous injections of a mix of recombinant Der p 1 and Der p 2 allergens. 7 days after the third injection of low dose of allergens, mice were challenged with a single massive dose of allergen. Mean and standard deviation of body temperatures of a set of ten mice is shown after challenge with either AllerDM (closed circles), vehicle (PBS, open circles) or the mix Der p 1 / Der p 2 (closed squares).
Figure 7 depicts the immunogenicity of AllerDM compared to Der p 1 / Der p 2 injections in Balb/c mice with Freund's adjuvant (panel A). AllerDM is able to induce specific IgGs recognizing the allergens Der p 1 and Der p 2 respectively up to levels comparable to immunization with the respective original allergens. AllerDM1.42, DM 2.4 and DM 2.10 contribute most to AllerDM immunogenicity, whereas individual mice do react to the other COPs present in AllerDM except for AllerDM2.9 (panel B).

### DETAILED DESCRIPTION OF THE INVENTION

The invention is described below by way of examples with reference to the following experimental procedures and results.

### Material and Methods

### Allergens

Purified natural Der p 1(NA-DP1-2) and natural Der p 2 (NA-DP2-2) were obtained from Indoor Biotechnologies Ltd, UK.

### Choice of Peptides and synthesis

The aim was to prevent the formation of stable tertiary structures of B cell epitopes, while presenting all T cell epitopes present within the Der p 1 and Der p 2 protein sequences presented below as SEQ ID NO: 27 (Der p 1) Swiss-Prot sequence P08176.2 and SEQ ID NO: 28 (Der p2) GenBank sequence AFJ68067.1 set out below. It should be noted that Der p 2 exists in multiple isotypes with Swiss Prot P49278.1 (SEQ ID NO: 29) described in previous patent application WO 2004-081028 (A2). There are four amino acid differences between the GenBank sequence AFJ68067.1 and the mature protein (without signal peptide) Swiss Prot P49278.1 sequence. The sequences also differ with the Swiss Prot P49278.1 sequence being shorter due to a single peptide being cleaved off its N-terminal end.

As a result, the following COPs which overlap along the Der p1 and Der p 2 sequences were selected, namely:

SEQ ID NO: 7
   ALLERDM1.7 (40AA) VDYWIVRNSWDTNWGDNGYGYFAANIDLMMIEEYPYVVIL

SEQ ID NO: 11
   ALLERDM1.72 (43AA) AQGVDYWIVRNSWDTNWGDNGYGYFAANIDLMMIEEYPYVVIL
SEQ ID NO: 12
   ALLERDM1.53 (37AA) ASQHGCHGDTIPRGIEYIQHNGVVQESYYRYVAREQS
SEQ ID NO: 13
   AllerDM1.54 (35AA) RYVAREQSCRRPNAQRFGISNYCQIYPPNVNKIRE
SEQ ID NO: 14
   AllerDM1.63 (35AA) PNVNKIREALAQTHSAIAVIIGIKDLDAFRHYDGR
SEQ ID NO: 15
   AllerDM1.64 (34AA) RHYDGRTIIQRDNGYQPNYHAVNIVGYSNAQGVD

SEQ ID NO: 18
   ALLERDM2.3 (25AA) DQVDVKDCANHEIKKVLVPGCHGSE
SEQ ID NO: 19
   ALLERDM2.4 (56AA) HGSEPCIIHRGKPFQLEALFEANQNSKTAKIEIKASIDGLEVDVPGIDPNACHYMK
SEQ ID NO: 20
   ALLERDM2.5 (56AA) HYMKCPLVKGQQYDIKYTWNVPKIAPKSENVVVTVKVLGDNGVLACAIATHAKIRD
SEQ ID NO: 21
   ALLERDM2.6 (31AA) DQVDVKDCANHEIKKVLVPGCHGSEPCIIHR

SEQ ID NO: 23
   ALLERDM2.8 (41AA) KYTWNVPKIAPKSENVVVTVKVLGDNGVLACAIATHAKIRD

SEQ ID NO: 25
   ALLERDM2.9 (21AA) KVLGDNGVLACAIATHAKIRD
SEQ ID NO: 26
   ALLERDM1.5a (32AA) AREQSCRRPNAQRFGISNYCQIYPPNVNKIRE

All COPs were synthesized by solid phase fmoc chemistry at research scale to allow determination of IgE binding. Preparative HPLC was used to obtain over 90% pure peptides which were lyophilized. Peptides were resuspended either in water at 2 mg/ml or at 10 to 20 mg per ml in DMSO (see Table 1) and frozen in aliquots.

### Competition ELISA

Purified Der p 1 or Der p 2 at 1.0 µg/ml were coated overnight on 96-well Nunc Maxisorp® immunoplates (Thermo Fisher Scientific Inc., Wohlen, Switzerland). After blocking with 1% BSA, either twenty-fold, thirty-fold or forty-fold up to 200- fold dilutions of patient serum were added depending on specific IgE content. Biotin Mouse anti-human mAb IgE at 5 µg/ml (BioLegend, San Diego, CA) were then added and antibodies were revealed with Streptavidin HRP (BD-Biosciences, San Diego, CA) and the substrate TMB. Sera of allergic patients were obtained from US and Europe (PlasmaLab International, Everett, WA, USA and Biomnis, Lyon, France) respectively) selected for positive CAP for D. pteronyssinus and clear signal over background and used to test for competition with the peptides. Serial dilutions of each set of COPs, namely for Der p 1 Set A, B, C or D and for Der p 2 mixes D, E or F at concentrations ranging from 10⁻⁵ to 10⁻¹⁰ M were pre-incubated with selected serums for 15 min. on ice. Serums were then incubated on nDer p 1 or nDer p 2 coated 96-well plates and residual IgE binding was determined as described above. nDer p 1 and nDer p 2 dilutions were used as control for inhibition.

### RBL degranulation

Degranulation assay was performed as described in [37]. RBL-703/21 cells transfected with human Fc epsilon RI receptor were plated in 96-well tissue culture plates (105 cells/well) overnight. Passive sensitization of RBL-703/21 cells was carried out with sera from patients with house dust mite allergy at 1:20 overnight. Unbound antibodies were removed by washing the cell layer twice in Hanks' balanced salt solution (Gibco, Life technologies) the next day. Degranulation of RBL cells was induced for 1h at 37°C by adding nDer p1, nDer p 2 or individual COPs and mixes at indicated concentrations diluted in Tyrode's buffer (130 mM NaCl, 5 mM KCl, 1.4 mM CaCl2, 1 mM MgCl2, 5.6 mM glucose, 10 mM HEPES pH7.4, 0.1% BSA). β-hexosaminidase activity was analyzed by incubating 30 µl of cell supernatant with 50 µl of 1.3mg/ml 4-Nitrophenyl N-acetyl-β-D-glucosaminide (Sigma) in citrate buffer (0.1M, pH 4.5) for 1 h at 37°C. The reaction was stopped by addition of 100 µl glycine buffer (0.2M glycine, 0.2M NaCl, pH 10.7) and optical densities (OD) were measured at 405 nm.

### Anaphylactic response in mice

Balb/c mice were sensitized by repeated injections of low doses (1 µg) of an allergen mix of equimolar Der p 1 and Der p 2 by 3 subcutaneous injections at 14 days interval. Mice were challenged with high doses of either mix of Der p 1 and Der p 2 (30µg/animal) or AllerDM COPs (30µg/animal) 7 days after the last injection. Anaphylactic symptoms were scored using a scale of 6 grades (0, no symptoms to 5, death) adapted from Sade et al. J Investig Allergol Clin Immunol; 17(6): 379- 385 (2007). Rectal temperature was monitored at 15 minutes intervals for 60 minutes after challenge using a digital thermometer. PBS was used as control for challenge leading.

### Immunogenicity of AllerDM

Der p 1, Der p 2 allergens and individual AllerDM COPs were injected i.p. in Balb/c mice. The allergens and peptides were given together with Complete Freund's adjuvant. Injections were repeated twice at a one month interval with Incomplete Freund's Adjuvant. Blood was collected from the retroorbital sinus 15 days after the last injection and serum prepared by standard method. Results are expressed as µg/ml specific IgG.

### Experimental Results

### Choice of peptides

### Der p 1

In order to select for products with lowered IgE binding, four sets of long (30-90 amino acids) contiguous overlapping peptides (COP) were devised encompassing the entire Der p 1 allergen (Swiss-Prot P08176.2, thus providing all possible T cell epitopes. A first set of three COPs, Derp1_Set A composed of AllerDM1.1, 1.2 and 1.3, SEQ ID 1, SEQ ID2 and SEQ ID 3 respectively, had already been proposed patent deposit "Allergen peptide fragments and use thereof' WO2004-081028(A2). Derp1_Set A COPs were synthesized, purified and assayed for IgE binding using competition ELISA. Residual IgE binding was observed with different sera from patients allergic to house dust mites to AllerDM1.2 and AllerDM1.3 but not AllerDM1.1.

A second set (Derp1_Set B) was devised taking into account potential IgE binding regions from the literature and tested for IgE binding by competition ELISA. Derp1_Set B, a mix of AllerDM1.4, 1.5, 1.6, 1.7, respectively SEQ ID 4, 5, 6 and 7 showed residual IgE reactivity. a. When using individual peptides, AllerDM1.4 and 1.7 showed no detectable IgE binding, whereas AllerDM1.5 and 1.6 were found to be responsible for the reactivity previously observed with AllerDM1.2 and 1.3 or complete Derp1_Set B.

AllerDM1.5 included two cysteines which might reform a disulfide bridge and recreate an IgE epitope. Thus, the C-terminal cysteine was removed, leading to AllerDM1.52 (SEQ ID 9) which unfortunately still bound IgE. For AllerDM1.6, shortening the C-terminal part had also no influence on IgE binding (AllerDM1.62, SEQ ID 10). Since the N-terminal end of AllerDM1.52 and the C-terminal end of AllerDM1.62 were modified, alternative peptides to AllerDM1.4 and 1.7 had to be devised in order to provide sufficient overlaps to ensure the presence of possible T cell terminal epitopes. AllerDM1.42 and 1.72 received 4 and 3 additional amino acids in the overlapping region (SEQ ID 8 and 11 respectively).The candidate Derp1_Set C including AllerDM1.52 and 1.62 showing residual IgE binding was thus discarded.

After modeling the 3D structure of Der p 1 and the synthesized peptides up to now, a further set, namely Derp1_SetD was prepared in which each of the IgE binding peptides AllerDM1.52 and 1.62 were further split. AllerDM1.52 was split in two peptides, namely AllerDM1.53 and 1.5a (SEQ ID 12 and 26 respectively) and AllerDM1.62 was split in AllerDM1.63 and 1.64 (SEQ ID 14 and 15) (Figure 1). These peptides finally did not show any detectable IgE binding, indicating that all possible IgE epitopes had been removed. Since 1.53 and 1.5a overlapped by only 5 amino acids, an extended version AllerDM1.54 (SEQ ID 13), overlapping by 8 amino acids, was synthesized and showed the same IgE non-binding properties as AllerDM1.5a. This finding indicates that varying the COPs end by 3 amino acids has no effect on IgE binding capacity. Having to split AllerDM1.62 in two pieces was unexpected regarding current knowledge, since the region R248 to R254 had not been previously retained as potentially binding IgE (see background of the invention). The final set of COPs issued from Der p 1 with no detectable IgE binding includes six peptides, namely AllerDM1.42, 1.53, 1.54, 1.63, 1.64 and 1.72. (SEQ ID 8, 12, 13, 14, 15 and 11 respectively).

### Der p 2

The first set of COPs was derived from previous patent application WO2004-081028(A2) (Der p 2 isotype P49278.1) (SEQ ID 29) and contained two peptides of equal length AllerDM2.1 and 2.2 (SEQ ID 16 and 17). This set of COPs (Derp2_SetA) showed low but detectable IgE binding in competition ELISA and AllerDM2.2 was found to bind IgE in direct ELISA. Derp2_SetB, composed of AllerDM2.3, 2.4 and 2.5 (SEQ ID 18, 19 and 20) was devised in order to prevent disulfide bridges responsible for two short loops known to play a role in the maintenance of the 3D structure of Der p 2. In parallel an alternative set (Derp2_SetC) was synthesized, where disulfide bridges were kept, namely AllerDM2.6, 2.7 and 2.8 (SEQ ID 21, 22 and 23). Both Sets B and C, showed residual IgE binding in competition ELISA. In SetB IgE binding was restricted to AllerDM2.5 as confirmed by both direct and competition ELISA. IgE binding to individual COPs from SetC proved to be multiple. AllerDM2.7, which covers the central part of Der p 2, showed residual IgE binding in competition ELISA, whereas AllerDM2.8 (C-terminal part of Der p 2) was found to bind IgE non-specifically IgE from sera from allergic and non-allergic donors. In addition, Derp2_SetC elicited weak but detectable response in RBL degranulation assay and was therefore discarded from further AllerDM development.

Surprisingly, Derp2_SetB (AllerDM2.3, 2.4 and 2.5), was able to induce basophil degranulation in an RBL assays whereas Derp2_SetA (Aller DM 2.1 and 2.2) did not (Figure 2). Furthermore, AllerDM2.4 and 2.5 in combination resulted in degranulation, whereas each individual peptide did not (Figure 2, Panel C). The same phenomenon was observed when combining AllerDM2.1 with AllerDM2.5. Since AllerDM2.1 and AllerDM2.4 did not bind IgE when tested alone in competitive ELISA, and since two IgE epitopes are required in addition to trigger degranulation, these results raised the possibility that some interaction between the two peptides AllerDM2.4 and 2.5 takes place in solution leading to partial reconstitution of the original 3D structure of the allergen, thus re-creating a second IgE epitope.

Analyzing Der p 2 crystal structures in view of the results above lead to propose that AllerDM2.4 and 2.5 may partly refold in solution due to the presence of antiparallel beta sheets and combine to re-create a second IgE binding site. Since the combination AllerDM 2.2 in combination with either 2.1 or 2.4 did not induce degranulation, S57 to C73 was added at the N-terminus of AllerDM2.5 in the next set of COPs leading to AllerDM2.10 (SEQ ID 24). In order to further reduce IgE binding potential, AllerDM2.10 was trimmed at its C-terminus to D113. To maintain a minimal overlap (5 amino acids) with AllerDM2.10, AllerDM2.9 (SEQ ID 25) had to be synthesized starting at K109, in order to include the complete Der p 2 sequence. Derp2_SetD (AllerDM2.3, 2.4, 2.10 and 2.9) and its individual components showed no residual IgE binding in competition ELISA and were not able to trigger basophil degranulation anymore. The final set of COPs issued from Der p 2 thus includes four peptides, namely AllerDM2.3, 2.4, 2.10 and 2.9 (SEQ ID 18, 19, 24 and 25 respectively).

A further finding indicated that IgE binding and basophil degranulation is related to partial reconstitution of original 3D structure in solution came from the use of denaturing salts. Adding denaturing salts, including guanidinium chloride or urea, strongly diminished IgE binding to AllerDM1.52, 1.62 and 2.2 (Figure 3). This indicates an alternative for product formulation to decrease potential immediate allergic reactions upon injection of allergen fragments.

### AllerDM

In order to further verify the absence of IgE binding the final mix of ten peptides derived from both Der p 1 and Der p 2, now called AllerDM, namely composed of combination of AllerDM1.42, 1.53, 1.54, 1.63, 1.64 and 1.72. (SEQ ID 8, 12, 13, 14, 15 and 11 respectively) with AllerDM2.3, 2.4, 2.10 and 2.9 (SEQ ID 18, 19, 24 and 25 respectively) was used in competition ELISA and RBL degranulation tests. As seen in Figure 4, AllerDM showed no detectable reactivity up to a concentration of 10⁻⁵M with a panel of 21 sera from patients allergic to house dust mites from both Europe and US. Controls using either Der p 1 or Der p 2 natural allergens showed expected competition in the range of 10⁻⁷ to 10⁻⁹ M. Reduced IgE binding allows to consider administering a higher dose of COPs in human compared to traditional SIT treatments.

RBL cells transfected with human Fc epsilon receptor I were found to bind human IgE and degranulate in presence of allergens [37]. AllerDM, up to 10⁻⁵M again, was unable to trigger basophil degranulation when pre-loading RBL cells with a panel of 18 different human serums of patients allergic to house dust mites (Figure 5 A and B). On the contrary, Der p 1 and Der p 2 were able to induce degranulation in combination with the same serums. The absence of degranulation of basophils indicates a potentially diminished risk of immediate allergic reaction upon application in human.

From these experiments it can be concluded that a preferred product composed of ten COPs to be called AllerDM will contain a combination of soluble peptides derived from both Der p 1 and Der p 2 proteins. The preferred product candidate will be composed of SEQ ID: 8, SEQ ID NO: 12, SEQ ID NO: 13, SEQ ID NO: 14, SEQ ID NO: 15, SEQ ID NO: 11, SEQ ID NO: 18, SEQ ID NO: 19, SEQ ID NO: 24 and SEQ ID NO: 25.

A further improvement of the invention includes splitting COPs in parts with improved purification and solubility properties. Also disclosed are homologs of the COPs AllerDM 1.42 to 1.72 and AllerDM2.3 to 2.10, by amino acid changes within each peptide to produce homologs thereof wherein the reactivity of the homologs to IgE antibodies of patients who are allergic to HDM is eliminated while that with the T lymphocytes is still retained. Further d are homologs of the COPs by shifting the limits of the COPs within the house dust mites allergens Der p 1 and Der p 2. Such homologs will result in products with equivalent profiles of non-detectable IgE binding and T lymphocyte activity. Such products will present the same potential for safety and efficacy in human as AllerDM and can be considered as equivalent in terms of chances for treatment, unless shown otherwise. Suitable homologs characterized by no reactivity to anti house dust mites IgE antibodies while maintaining reactivity to T lymphocytes may be identified by the methods described herein

**Table I: Solubility Characteristics of COPs**

| COP | MW (Da) | Solvent | Conc. (mg/ml) |
|---|---|---|---|
| AllerDM1.1 | 8629.6 | H₂O (after addition of HCl and NaOH and citrate buffer pH5.5) | 1.0 |
| AllerDM1.2 | 9841.0 | H₂O (precipitates when added to Citrate buffer or PBS at 2x10⁻⁵M) | 2.0 |
| AllerDM1.3 | 9908.0 | DMSO | 0.5 |
| AllerDM1.4 | 7040.8 | H₂O (pH adjusted with HCl and NaOH) Difficult pH8 | 1.0 |
| AllerDM1.5 | 8470.4 | H₂O | 1.7 |
| AllerDM1.6 | 8314.2 | H₂O (after addition of HCl) | 0.8 |
| AllerDM1.7 | 4807.3 | H₂O | 1.0 |
| AllerDM1.42 | 7450.2 | 7 M guanidinium chloride | 30.0 |
| AllerDM1.52 | 7469.3 | H₂O | 2.3 |
| AllerDM1.62 | 6992.7 | H₂O | 2.5 |
| AllerDM1.72 | 5063.6 | DMSO | 12.0 |
| AllerDM1.53 | 4249.6 | H₂O | 4.0 |
| AllerDM1.54 | 4227.7 | H₂O | 2.0 |
| AllerDM1.63 | 3903.4 | H₂O | 2.0 |
| AllerDM1.64 | 3892.1 | H₂O | 4.0 |
| AllerDM1.5a | 3809.3 | H₂O | 4.7 |
| AllerDM2.1 | 7791.8 | H₂O (after addition of HCl, citrate buffer pH5) | 1.0 |
| AllerDM2.2 | 7940.2 | H₂O | 1.7 |
| AllerDM2.3 | 2721.0 | H₂O | 3.0 |
| AllerDM2.4 | 6162.0 | H₂O | 4.2 |
| AllerDM2.5 | 6225.3 | H₂O | 1.5 |
| AllerDM2.6 | 3440.9 | H₂O | 3.0 |
| AllerDM2.7 | 8414.7 | H₂O | 3.5 |
| AllerDM2.8 | 4420.2 | H₂O | 3.5 |
| AllerDM2.9 | 2165.5 | H₂O | 1.0 |
| AllerDM2.10 | 6286.2 | H₂O (after acidification with HCl and neutralization with NaOH) | 2.0 |

### Sensitization and challenge

Balb/c mice were sensitized by repeated subcutaneous injections of a mix of Der p 1 and Der p 2 allergens (1µg/ml). 7 days after the last injection mice were challenged with a single massive dose of allergens (Der p 1 and Der p 2 at 30µg/ml) or AllerDM (30µg/ml). Anaphylactic shock in mice has been associated with body temperature drop. Controls and AllerDM challenges resulted in essentially no temperature drop, whereas the mix Der p 1 / Der p 2 induced a marked decrease in rectal temperature (Figure 6). These results show that AllerDM differs from its parent allergens by a lack of challenging activity, indicating that AllerDM does not trigger an anaphylactic shock in Der p 1 / Der p 2 sensitized mice.

### Immunisation

AllerDM and Der p 1/Der p 2 were injected in Balb/c mice with Freund's adjuvant. IgG levels were measured after intraperitoneal injection (Figure 7). IgGs raised by the AllerDM mix recognize natural Der p 1 and Der p 2 to almost the same extend as the original allergens themselves (Figure 7, panel A). In separate experiments, the presence of IgGs against each individual COP was detected in sera from mice immunized with AllerDM (Figure 7, panel B) showing that each COP can contribute to the overall immunogenicity, albeit to different extent.

### Variations in the invention

Also disclosed are homologs of the AllerDM1.42 to DM2.10 COPs, by amino acid changes within each peptide to produce homologs thereof wherein the reactivity of the homologs to IgE antibodies of patients who are allergic to house dust mite allergens is eliminated while that with the T lymphocytes is still retained. Further disclosed are homologs of the COPs by shifting the limits of the COPs within the house dust mite allergens Der p 1 and Der p 2. Such homologs will result in products with equivalent profiles of non-detectable IgE binding and T lymphocyte activity. Such products will present the same potential for safety and efficacy in human as AllerDM and can be considered as equivalent in terms of chances for treatment, unless shown otherwise. Suitable homologs characterized by no reactivity to anti house dust mite IgE antibodies while maintaining reactivity to T lymphocytes may be identified by the methods described herein. Also disclosed are sets of COPs with reduced or eliminated IgE reactivity which retain T lymphocyte reactivity but which are also soluble and/or which show improved synthesis and purification.

Numerous modifications and variations in the practice of the invention are expected to occur to those skilled in the art upon consideration of the present disclosure. Consequently, the only limitations which should be placed upon the scope of the invention are those which appear in the appended claims.

### References:

[1] Settipane RA. Demographics and epidemiology of allergic and nonallergic rhinitis. Allergy Asthma Proc. 2001;22:185-9.
[2] Bousquet J, Bullinger M, Fayol C, Marquis P, Valentin B, Burtin B. Assessment of quality of life in patients with perennial allergic rhinitis with the French version of the SF-36 Health Status Questionnaire. J Allergy Clin Immunol. 1994;94:182-8.
[3] Thomas WR. Geography of house dust mite allergens. Asian Pacific journal of allergy and immunology / launched by the Allergy and Immunology Society of Thailand. 2010;28:211-24.
[4] Weghofer M, Thomas WR, Kronqvist M, Mari A, Purohit A, Pauli G, et al. Variability of IgE reactivity profiles among European mite allergic patients. European journal of clinical investigation. 2008;38:959-65.
[5] Taketomi EA, Silva DA, Sopelete MC, Gervasio AM, Alves R, Sung SJ. Differential IgE reactivity to Der p 1 and Der p 2 allergens of Dermatophagoides pteronyssinus in mite-sensitized patients. Journal of investigational allergology & clinical immunology : official organ of the International Association of Asthmology. 2006;16:104-9.
[6] Hales BJ, Martin AC, Pearce LJ, Laing IA, Hayden CM, Goldblatt J, et al. IgE and IgG anti-house dust mite specificities in allergic disease. J Allergy Clin Immunol. 2006;118:361-7.
[7] Smith WA, Hales BJ, Jarnicki AG, Thomas WR. Allergens of wild house dust mites: environmental Der p 1 and Der p 2 sequence polymorphisms. J Allergy Clin Immunol. 2001;107:985-92.
[8] Piboonpocanun S, Malainual N, Jirapongsananuruk O, Vichyanond P, Thomas WR. Genetic polymorphisms of major house dust mite allergens. Clin Exp Allergy. 2006;36:510-6.
[9] Jeannin P, Pestel J, Bossus M, Lassalle P, Tartar A, Tonnel AB. Comparative analysis of biological activities of Der p I-derived peptides on Fc epsilon receptor-bearing cells from Dermatophagoides pteronyssinus-sensitive patients. Clinical and experimental immunology. 1993;92:133-8.
[10] Jarnicki AG, Tsuji T, Thomas WR. Inhibition of mucosal and systemic T(h)2-type immune responses by intranasal peptides containing a dominant T cell epitope of the allergen Der p 1. International immunology. 2001;13:1223-31.
[11] Greene WK, Thomas WR. IgE binding structures of the major house dust mite allergen Der p I. Molecular immunology. 1992;29:257-62.
[12] Greene WK, Chua KY, Stewart GA, Thomas WR. Antigenic analysis of group I house dust mite allergens using random fragments of Der p I expressed by recombinant DNA libraries. International archives of allergy and applied immunology. 1990;92:30-8.
[13] Chruszcz M, Pomes A, Glesner J, Vailes LD, Osinski T, Porebski PJ, et al. Molecular determinants for antibody binding on group 1 house dust mite allergens. The Journal of biological chemistry. 2012;287:7388-98.
[14] Dai YC, Chuang WJ, Chua KY, Shieh CC, Wang JY. Epitope mapping and structural analysis of the anti-Der p 1 monoclonal antibody: insight into therapeutic potential. Journal of molecular medicine. 2011;89:701-12.
[15] Christensen LH, Riise E, Bang L, Zhang C, Lund K. Isoallergen variations contribute to the overall complexity of effector cell degranulation: effect mediated through differentiated IgE affinity. J Immunol. 2010;184:4966-72.
[16] Hales BJ, Hazell LA, Smith W, Thomas WR. Genetic variation of Der p 2 allergens: effects on T cell responses and immunoglobulin E binding. Clin Exp Allergy. 2002;32:1461-7.
[17] Mueller GA, Smith AM, Chapman MD, Rule GS, Benjamin DC. Hydrogen exchange nuclear magnetic resonance spectroscopy mapping of antibody epitopes on the house dust mite allergen Der p 2. The Journal of biological chemistry. 2001;276:9359-65.
[18] Szalai K, Fuhrmann J, Pavkov T, Scheidl M, Wallmann J, Bramswig KH, et al. Mimotopes identify conformational B-cell epitopes on the two major house dust mite allergens Der p 1 and Der p 2. Molecular immunology. 2008;45:1308-17.
[19] Drachenberg KJ, Heinzkill M, Urban E, Woroniecki SR. Efficacy and tolerability of short-term specific immunotherapy with pollen allergoids adjuvanted by monophosphoryl lipid A (MPL) for children and adolescents. Allergol Immunopathol (Madr). 2003;31:270-7.
[20] Dam Petersen K G-HD, Kjaergaard S, Dahl R. Clinical and patient based evaluation of immunotherapy for grass pollen and mite allergy. Allergol Immunopathol (Madr). 2005;33:264-9.
[21] Birnbaum J, Ramadour M, Magnan A, Vervloet D. Hymenoptera ultra-rush venom immunotherapy (210 min): a safety study and risk factors. Clin Exp Allergy. 2003;33:58-64.
[22] Drachenberg KJ, Proll S, Urban E, Woroniecki SR. Single-course specific immunotherapy with mixed pollen allergoids: results of a multi-centre study. Allergol Immunopathol (Madr). 2003;31:77-82.
[23] Hartl A, Hochreiter R, Stepanoska T, Ferreira F, Thalhamer J. Characterization of the protective and therapeutic efficiency of a DNA vaccine encoding the major birch pollen allergen Bet v 1a. Allergy. 2004;59:65-73.
[24] Martinez Gomez JM, Fischer S, Csaba N, Kundig TM, Merkle HP, Gander B, et al. A protective allergy vaccine based on CpG- and protamine-containing PLGA microparticles. Pharm Res. 2007;24:1927-35.
[25] Pauli G, Larsen TH, Rak S, Horak F, Pastorello E, Valenta R, et al. Efficacy of recombinant birch pollen vaccine for the treatment of birch-allergic rhinoconjunctivitis. J Allergy Clin Immunol. 2008;122:951-60.
[26] Walgraffe D, Matteotti C, El Bakkoury M, Garcia L, Marchand C, Bullens D, et al. A hypoallergenic variant of Der p 1 as a candidate for mite allergy vaccines. J Allergy Clin Immunol. 2009;123:1150-6.
[27] van Oort E, de Heer PG, van Leeuwen WA, Derksen NI, Muller M, Huveneers S, et al. Maturation of Pichia pastoris-derived recombinant pro-Der p 1 induced by deglycosylation and by the natural cysteine protease Der p 1 from house dust mite. European journal of biochemistry / FEBS. 2002;269:671-9.
[28] Asturias JA, Ibarrola I, Arilla MC, Vidal C, Ferrer A, Gamboa PM, et al. Engineering of major house dust mite allergens Der p 1 and Der p 2 for allergen-specific immunotherapy. Clin Exp Allergy. 2009;39:1088-98.
[29] Chen KW, Blatt K, Thomas WR, Swoboda I, Valent P, Valenta R, et al. Hypoallergenic Der p 1/Der p 2 combination vaccines for immunotherapy of house dust mite allergy. J Allergy Clin Immunol. 2012;130:435-43 e4.
[30] Pulsawat P, Piboonpocanun S, Sirivichayakul S, Buranapraditkun S, Jacquet A, Shimada M, et al. Production and immunogenicity of hypoallergenic codon-optimized DNA vaccine encoding mature Der p 1 allergen. Journal of investigational allergology & clinical immunology : official organ of the International Association of Asthmology. 2010;20:582-90.
[31] Pulsawat P, Pitakpolrat P, Prompetchara E, Kaewamatawong T, Techakriengkrai N, Sirivichayakul S, et al. Optimization of a Der p 2-based prophylactic DNA vaccine against house dust mite allergy. Immunology letters. 2013;151:23-30.
[32] Patel D, Couroux P, Hickey P, Salapatek AM, Laidler P, Larche M, et al. Fel d 1-derived peptide antigen desensitization shows a persistent treatment effect 1 year after the start of dosing: A randomized, placebo-controlled study. J Allergy Clin Immunol. 2013;131:103-9 e7.
[33] Jahn-Schmid B, Radakovics A, Luttkopf D, Scheurer S, Vieths S, Ebner C, et al. Bet v 1142-156 is the dominant T-cell epitope of the major birch pollen allergen and important for cross-reactivity with Bet v 1-related food allergens. J Allergy Clin Immunol. 2005;116:213-9.
[34] Niederberger V, Horak F, Vrtala S, Spitzauer S, Krauth MT, Valent P, et al. Vaccination with genetically engineered allergens prevents progression of allergic disease. Proc Natl Acad Sci U S A. 2004;101 Suppl 2:14677-82.
[35] Purohit A, Niederberger V, Kronqvist M, Horak F, Gronneberg R, Suck R, et al. Clinical effects of immunotherapy with genetically modified recombinant birch pollen Bet v 1 derivatives. Clin Exp Allergy. 2008.
[36] Fellrath JM, Kettner A, Dufour N, Frigerio C, Schneeberger D, Leimgruber A, et al. Allergen-specific T-cell tolerance induction with allergen-derived long synthetic peptides: results of a phase I trial. J Allergy Clin Immunol. 2003;111:854-61.
[37] Vogel L, Luttkopf D, Hatahet L, Haustein D, Vieths S. Development of a functional in vitro assay as a novel tool for the standardization of allergen extracts in the human system. Allergy. 2005;60:1021-8.

Numerous modifications and variations in the practice of the invention are expected to occur to those skilled in the art upon consideration of the presently preferred embodiments thereof. Consequently, the only limitations which should be placed upon the scope of the invention are those which appear in the appended claims.

## Claims

1. A composition comprising six contiguous overlapping peptides, wherein
- the first peptide consists of the sequence from amino acid T99 to amino acid G168 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acid A164 to amino acid S200 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid R193 to amino acid E227 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the fourth peptide consists of the sequence from amino acidP220 to amino acid R254 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the fifth peptide consists of the sequence from amino acid R249 to amino acid D282 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained, and
- the sixth peptide consists of the sequence from amino acid A278 to amino acid L320 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained.

2. A composition comprising four contiguous overlapping peptides, wherein
- the first peptide consists of the sequence from amino acid D1 to amino acid E25 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acid H22 to amino acid K77 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid S57 to amino acid D113 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained, and
- the fourth peptide consists of the sequence from amino acid K109 to amino acid D129 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained.

3. A composition comprising a plurality of Der p1 contiguous overlapping peptides and a plurality of Der p2 contiguous overlapping peptides, wherein said plurality of Der p1 contiguous overlapping peptides are six peptides, wherein
- the first peptide consists of the sequence from amino acid T99 to amino acid G168 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acids A164 to amino acid S200 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid R193 to amino acid E227 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the fourth peptide consists of the sequence from amino acid P220 to amino acid R254 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the fifth peptide consists of the sequence from amino acid R249 to amino acid D282 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained,
- the sixth peptide consists of the sequence from amino acid A278 to amino acid L320 of a polypeptide having SEQ ID NO:27 wherein the reactivity of said peptide to IgE antibodies of subjects who are allergic to house dust mites is eliminated while the reactivity with the T lymphocytes from subjects who are allergic to house dust mites is retained;
and wherein the plurality of Der p2 contiguous overlapping peptides are four peptides, wherein
- the first polypeptide consists of the sequence from amino acid D1 to amino acid E25 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acid H22 to amino acid K77 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid S57 to amino acid D113 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained, and
- the fourth peptide consists of the sequence from amino acid K109 to amino acid D129 of a polypeptide having SEQ ID NO:28 wherein the reactivity of said peptide to IgE antibodies of subjects who are allergic to house dust mites is eliminated while the reactivity with the T lymphocytes from subjects who are allergic to house dust mites is retained.

4. A combination of allergens for use in a method of specific immunotherapy against house dust mites allergies in a patient, wherein said combination of allergens comprises ten peptides, wherein
- the first peptide consists of the sequence from amino acid T99 to amino acid G168 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the second peptide consists of the sequence from amino acid A164 to amino acid S200 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the third peptide consists of the sequence from amino acid R193 to amino acid E227 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the fourth peptide consists of the sequence from amino acid P220 to amino acid R254 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the fifth peptide consists of the sequence from amino acid R249 to amino acid D282 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the sixth peptide consists of the sequence from amino acid A278 to amino acid L320 of a polypeptide having SEQ ID NO: 27 wherein the reactivity of said peptide to IgE antibodies of subjects who are allergic to house dust mites is eliminated while the reactivity with the T lymphocytes from subjects who are allergic to house dust mites is retained,
- the seventh peptide consists of the sequence from amino acid D1 to amino acid E25 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained,
- the eighth peptide consists of the sequence from amino acid H22 to amino acid K77 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained,
- the ninth peptide consists of the sequence from amino acid S57 to amino acid D113 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained, and
- the tenth peptide consists of the sequence from amino acid K109 to amino acid D129 of a polypeptide having SEQ ID NO: 28 wherein the reactivity of said peptide to IgE antibodies of subjects who are allergic to house dust mites is eliminated while the reactivity with the T lymphocytes from subjects who are allergic to house dust mites is retained,
and wherein said combination of ten allergens is administered to a patient in need thereof.

5. The combination of allergens for use according to claim 4, wherein the peptides are administered to the patient using intradermal injection, subcutaneous injection, intramuscular injection, intravenous injection, transdermal, intranasal, oral, sublingual, intraocular, or intrathecal techniques.

6. The combination of allergens for use according to claim 4 or 5, wherein
(i) the peptides are administered in the presence of a denaturing or chaotropic agent, preferably wherein the chaotropic agent is guanidinium chloride;
(ii) the allergens are provided in dry powdered form;
(iii) the allergens further comprise a pharmaceutically acceptable carrier or diluent; and/or
(iv) the allergens further comprise an adjuvant, preferably wherein the adjuvant is aluminium hydroxide.

## Patentansprüche

1. Eine Zusammensetzung, umfassend sechs zusammenhängende, überlappende Peptide, wobei
- das erste Peptid aus der Sequenz von Aminosäure T99 bis Aminosäure G168 eines Polypeptids mit SEQ ID NO:27 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das zweite Peptid aus der Sequenz von Aminosäure A164 bis Aminosäure S200 eines Polypeptids mit SEQ ID NO:27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das dritte Peptid aus der Sequenz von Aminosäure R193 bis Aminosäure E227 eines Polypeptids mit SEQ ID NO:27 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das vierte Peptid aus der Sequenz von Aminosäure P220 bis Aminosäure R254 eines Polypeptids mit SEQ ID NO:27 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das fünfte Peptid aus der Sequenz von Aminosäure R249 bis Aminosäure D282 eines Polypeptids mit SEQ ID NO:27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist, und
- das sechste Peptid aus der Sequenz von Aminosäure A278 bis Aminosäure L320 eines Polypeptids mit SEQ ID NO:27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist.

2. Eine Zusammensetzung, umfassend vier zusammenhängende, überlappende Peptide, wobei
- das erste Peptid aus der Sequenz von Aminosäure D1 bis Aminosäure E25 eines Polypeptids mit SEQ ID NO:28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das zweite Peptid aus der Sequenz von Aminosäure H22 bis Aminosäure K77 eines Polypeptids mit SEQ ID NO:28 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das dritte Peptid aus der Sequenz von Aminosäure S57 bis Aminosäure D113 eines Polypeptids mit SEQ ID NO:28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist, und
- das vierte Peptid aus der Sequenz von Aminosäure K109 bis Aminosäure D129 eines Polypeptids mit SEQ ID NO:28 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist.

3. Eine Zusammensetzung, umfassend eine Vielzahl von Der p1 zusammenhängenden, überlappenden Peptiden und eine Vielzahl von Der p2 zusammenhängenden, überlappenden Peptiden, wobei die Vielzahl von Der p1 zusammenhängenden, überlappenden Peptiden sechs Peptide sind, wobei
- das erste Peptid aus der Sequenz von Aminosäure T99 bis Aminosäure G168 eines Polypeptids mit SEQ ID NO:27 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das zweite Peptid aus der Sequenz von Aminosäuren A164 bis Aminosäure S200 eines Polypeptids mit SEQ ID NO:27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das dritte Peptid aus der Sequenz von Aminosäure R193 bis Aminosäure E227 eines Polypeptids mit SEQ ID NO:27 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das vierte Peptid aus der Sequenz von Aminosäure P220 bis Aminosäure R254 eines Polypeptids mit SEQ ID NO:27 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das fünfte Peptid aus der Sequenz von Aminosäure R249 bis Aminosäure D282 eines Polypeptids mit SEQ ID NO:27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das sechste Peptid aus der Sequenz von Aminosäure A278 bis Aminosäure L320 eines Polypeptids mit SEQ ID NO:27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist;
und wobei die Vielzahl von Der p2 zusammenhängenden, überlappenden Peptiden vier Peptide sind, wobei
- das erste Peptid aus der Sequenz von Aminosäure D1 bis Aminosäure E25 eines Polypeptids mit SEQ ID NO:28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das zweite Peptid aus der Sequenz von Aminosäure H22 bis Aminosäure K77 eines Polypeptids mit SEQ ID NO:28 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist,
- das dritte Peptid aus der Sequenz von Aminosäure S57 bis Aminosäure D113 eines Polypeptids mit SEQ ID NO:28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist, und
- das vierte Peptid aus der Sequenz von Aminosäure K109 bis Aminosäure D129 eines Polypeptids mit SEQ ID NO:28 besteht, wobei die Reaktivität des Peptids auf IgE-Antikörper von Subjekten, die allergisch gegen Hausstaubmilben sind, eliminiert ist, während die Reaktivität mit den T-Lymphozyten von Subjekten, die allergisch gegen Hausstaubmilben sind, erhalten ist.

4. Eine Kombination von Allergenen zur Verwendung in einem Verfahren zur spezifischen Immuntherapie gegen Hausstaubmilbenallergien bei einem Patienten, wobei die Kombination von Allergenen zehn Peptide umfasst, wobei
- das erste Peptid aus der Sequenz von Aminosäure T99 bis Aminosäure G168 eines Polypeptids mit SEQ ID NO: 27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das zweite Peptid aus der Sequenz von Aminosäure A164 bis Aminosäure S200 eines Polypeptids mit SEQ ID NO: 27 besteht, wobei die Reaktivität des Peptids gegenüber IgE-Antikörpern von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das dritte Peptid aus der Sequenz von Aminosäure R193 bis Aminosäure E227 eines Polypeptids mit SEQ ID NO: 27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das vierte Peptid aus der Sequenz von Aminosäure P220 bis Aminosäure R254 eines Polypeptids mit SEQ ID NO: 27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das fünfte Peptid aus der Sequenz von Aminosäure R249 bis Aminosäure D282 eines Polypeptids mit SEQ ID NO: 27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das sechste Peptid aus der Sequenz von Aminosäure A278 bis Aminosäure L3220 eines Polypeptids mit SEQ ID NO: 27 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das siebte Peptid aus der Sequenz von Aminosäure D1 bis Aminosäure E25 eines Polypeptids mit SEQ ID NO: 28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das achte Peptid aus der Sequenz von Aminosäure H22 bis Aminosäure K77 eines Polypeptids mit SEQ ID NO: 28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
- das neunte Peptid aus der Sequenz von Aminosäure S57 bis Aminosäure D113 eines Polypeptids mit SEQ ID NO: 28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist, und
- das zehnte Peptid aus der Sequenz von Aminosäure K109 bis Aminosäure D129 eines Polypeptids mit SEQ ID NO: 28 besteht, wobei die Reaktivität des Peptids auf IgE-Antikörper von Subjekten, die gegen Hausstaubmilben allergisch sind, eliminiert ist, während die Reaktivität mit den T-Lymphozyten von Subjekten, die gegen Hausstaubmilben allergisch sind, erhalten ist,
und wobei die Kombination von zehn Allergenen einem Patienten, der sie benötigt, verabreicht wird.

5. Die Kombination von Allergenen zur Verwendung nach Anspruch 4, wobei die Peptide dem Patienten mittels intradermaler Injektion, subkutaner Injektion, intramuskulärer Injektion, intravenöser Injektion, transdermaler, intranasaler, oraler, sublingualer, intraokularer oder intrathekaler Techniken verabreicht werden.

6. Die Kombination von Allergenen zur Verwendung nach Anspruch 4 oder 5, wobei
(i) die Peptide in Gegenwart eines denaturierenden oder chaotropen Mittels verabreicht werden, wobei das chaotrope Mittel vorzugsweise Guanidiniumchlorid ist;
(ii) die Allergene in trockener Pulverform bereitgestellt werden;
(iii) die Allergene ferner einen pharmazeutisch annehmbaren Träger oder ein pharmazeutisch annehmbares Verdünnungsmittel umfassen; und/oder
(iv) die Allergene ferner ein Adjuvans umfassen, wobei das Adjuvans vorzugsweise Aluminiumhydroxid ist.

## Revendications

1. Composition comprenant six peptides contigus se chevauchant, dans laquelle
- le premier peptide consiste en la séquence s'étendant de l'acide aminé T99 à l'acide aminé G168 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le deuxième peptide consiste en la séquence s'étendant de l'acide aminé A164 à l'acide aminé S200 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le troisième peptide consiste en la séquence s'étendant de l'acide aminé R193 à l'acide aminé E227 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le quatrième peptide consiste en la séquence s'étendant de l'acide aminé P220 à l'acide aminé R254 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le cinquième peptide consiste en la séquence s'étendant de l'acide aminé R249 à l'acide aminé D282 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée, et
- le sixième peptide consiste en la séquence s'étendant de l'acide aminé A278 à l'acide aminé L320 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée.

2. Composition comprenant quatre peptides contigus se chevauchant, dans laquelle
- le premier peptide consiste en la séquence s'étendant de l'acide aminé D1 à l'acide aminé E25 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le deuxième peptide consiste en la séquence s'étendant de l'acide aminé H22 à l'acide aminé K77 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le troisième peptide consiste en la séquence s'étendant de l'acide aminé S57 à l'acide aminé D113 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée, et
- le quatrième peptide consiste en la séquence s'étendant de l'acide aminé K109 à l'acide aminé D129 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée.

3. Composition comprenant une pluralité de peptides contigus se chevauchant Der p1 et une pluralité de peptides contigus se chevauchant Der p2, dans laquelle ladite pluralité de peptides contigus se chevauchant Der p1 consiste en six peptides, où
- le premier peptide consiste en la séquence s'étendant de l'acide aminé T99 à l'acide aminé G168 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le deuxième peptide consiste en la séquence s'étendant de l'acide aminé A164 à l'acide aminé S200 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le troisième peptide consiste en la séquence s'étendant de l'acide aminé R193 à l'acide aminé E227 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le quatrième peptide consiste en la séquence s'étendant de l'acide aminé P220 à l'acide aminé R254 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le cinquième peptide consiste en la séquence s'étendant de l'acide aminé R249 à l'acide aminé D282 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le sixième peptide consiste en la séquence s'étendant de l'acide aminé A278 à l'acide aminé L320 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée.
et dans laquelle la pluralité de peptides contigus se chevauchant Der p2 consiste en quatre peptides, où
- le premier peptide consiste en la séquence s'étendant de l'acide aminé D1 à l'acide aminé E25 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le deuxième peptide consiste en la séquence s'étendant de l'acide aminé H22 à l'acide aminé K77 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le troisième peptide consiste en la séquence s'étendant de l'acide aminé S57 à l'acide aminé D113 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le quatrième peptide consiste en la séquence s'étendant de l'acide aminé K109 à l'acide aminé D129 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée.

4. Association d'allergènes destinée à être utilisée dans un procédé d'immunothérapie spécifique contre des allergies aux acariens de la poussière de maison chez un patient, dans laquelle ladite association d'allergènes comprend dix peptides, où
- le premier peptide consiste en la séquence s'étendant de l'acide aminé T99 à l'acide aminé G168 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le deuxième peptide consiste en la séquence s'étendant de l'acide aminé A164 à l'acide aminé S200 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le troisième peptide consiste en la séquence s'étendant de l'acide aminé R193 à l'acide aminé E227 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le quatrième peptide consiste en la séquence s'étendant de l'acide aminé P220 à l'acide aminé R254 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le cinquième peptide consiste en la séquence s'étendant de l'acide aminé R249 à l'acide aminé D282 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le sixième peptide consiste en la séquence s'étendant de l'acide aminé A278 à l'acide aminé L320 d'un polypeptide ayant la séquence SEQ ID NO: 27, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le septième peptide consiste en la séquence s'étendant de l'acide aminé D1 à l'acide aminé E25 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le huitième peptide consiste en la séquence s'étendant de l'acide aminé H22 à l'acide aminé K77 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
- le neuvième peptide consiste en la séquence s'étendant de l'acide aminé S57 à l'acide aminé D113 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée, et
- le dixième peptide consiste en la séquence s'étendant de l'acide aminé K109 à l'acide aminé D129 d'un polypeptide ayant la séquence SEQ ID NO: 28, la réactivité dudit peptide vis-à-vis d'anticorps IgE de sujets qui sont allergiques aux acariens de la poussière de maison étant éliminée alors que la réactivité avec les lymphocytes T provenant de sujets qui sont allergiques aux acariens de la poussière de maison est conservée,
et dans laquelle ladite association de dix allergènes est administrée à un patient en ayant besoin.

5. Association d'allergènes destinée à être utilisée selon la revendication 4, dans laquelle on administre les peptides au patient en utilisant l'injection intradermique, l'injection sous-cutanée, l'injection intramusculaire, l'injection intraveineuse, les techniques transdermique, intranasale, orale, sublinguale, intraoculaire, ou intrathécale.

6. Association d'allergènes destinée à être utilisée selon la revendication 4 ou 5, dans laquelle
(i) les peptides sont administrés en la présence d'un agent dénaturant ou chaotrope, de préférence l'agent chaotrope étant le chlorure de guanidinium ;
(ii) les allergènes sont fournis sous forme de poudre sèche ;
(iii) les allergènes comprennent en outre un véhicule ou diluant pharmaceutiquement acceptable ; et/ou
(iv) les allergènes comprennent en outre un adjuvant, de préférence l'adjuvant étant l'hydroxyde d'aluminium.
